# EUROPEAN PATENT APPLICATION

(11) **EP 4 478 049 A2**
(43) Date of publication of application: **18.12.2024**
(21) Application number: 24208208.9
(22) Date of filing: 03.08.2017
(51) Int. Cl.: G01N 33/50

(54) **CANCER MODELING PLATFORMS AND METHODS OF USING THE SAME**

(30) Priority: 03.08.2016 US 201662370320 P; 20.06.2017 US 201762522313 P
(62) Divisional of application: 17837676.0
(71) Applicant: Wake Forest University Health Sciences, Winston-Salem, NC 27157 (US)
(72) Inventor: Skardal, Aleksander, Clemmons NC 27012 (US); Votanopoulos, Konstantinos, Winston-Salem 27106 (US)
(74) Representative: Marks & Clerk LLP

(57) **Abstract**

Described herein are *in vitro* cell constructs (or "organoids") useful as a tumor model, the constructs comprising live tumor cells from a subject. In some embodiments, provided are *in vitro* cell constructs comprising: *(a)* a core comprised of live tumor cells; and *(b)* a shell surrounding *(e.g.,* encapsulating) the core, the shell comprised of live benign cells (*e.g.,* tissue cells, benign or differentiated tumor cells, etc.). Also described herein are methods of making and using such constructs. Further provided are devices useful for evaluating tumor cells *in vitro,* comprising: *(a)* a microfluidic device having a chamber, and a channel in fluid communication with the chamber; *(b)* a live tumor cell construct (*e.g.,* an organoid) in the chamber; *(c)* a growth media in the chamber and the channel; *(d)* a pump operatively associated with the chamber and channel and configured for circulating the media from the chamber through the channel and back to the chamber; *(e)* a microporous membrane (*e.g.,* a TRANSWELL^{®} microporous membrane) in the channel and positioned so that the media flows therethrough.

## Description

### Related Application Information

This application claims the benefit of U.S. Provisional Patent Application Serial No. 62/370,320, filed August 3, 2016, and U.S. Provisional Patent Application Serial No. 62/522,313, filed June 20, 2017, the disclosure of each of which are incorporated herein by reference in their entirety.

### Statement of Government Support

This invention was made with government support under grant numbers 1U54TR001362-01 and 5UL1TR001420-03 awarded by the National Institutes of Health. The government has certain rights in this invention.

### Field of the Invention

This application relates to *in vitro* cell constructs (or "organoids") that may be used as a tumor model along with methods for making and using the same.

### Background

Precision medicine shows incredible promise for improving treatment in cancer patients. Currently, genetic profiling can be performed on tumor biopsies to narrow down drug choices based on key mutations, but choosing the absolute best drug for a particular patient remains difficult. Screening drug choices with tumor models made with patient's own cells could supplement genetic screening to determine the most efficacious drugs. These patient specific cancer models would yield personalized, predictive data about the effect of drugs on the growth of tumors and their ability to metastasize.

### Summary of the Invention

A first aspect of the invention is an *in vitro* cell construct (or "organoid") useful as a tumor model, comprising: *(a)* a core comprised of live tumor cells; and *(b)* a shell surrounding (*e.g.*, encapsulating) the core, the shell comprised of live benign cells (*e.g.*, tissue cells, benign or differentiated tumor cells, etc.). The live tumor cells and/or live benign cells may be obtained from a subject, such as, for example, from a biopsy (e.g., a tumor biopsy and/or tissue biopsy) taken from the subject.

A second aspect of the invention is an *in vitro* cell construct (or "organoid") useful as a tumor model, comprising live tumor cells from a subject. In some embodiments, the live tumor cells are from a biopsy (e.g., a tumor biopsy) taken from the subject.

A further aspect of the invention is a device useful for evaluating tumor cells *in vitro* (e.g., useful for evaluating metastasis of tumor cells *in vitro*, for evaluating tumor cell migration and/or invasion *in vitro*, for evaluating growth of a construct comprising tumor cells *in vitro*, and/or for evaluating response to a compound of interest, such as, e.g., a drug or prodrug), comprising: *(a)* a microfluidic device having a chamber, and a channel in fluid communication with the chamber; *(b)* a live tumor cell construct (*e.g.*, an organoid) in the chamber; *(c)* a growth media in the chamber and the channel; *(d)* a pump operatively associated with the chamber and channel and configured for circulating the media from the chamber through the channel and back to the chamber; *(e)* a microporous membrane (*e.g.*, a TRANSWELL^{®} microporous membrane) in the channel and positioned so that the media flows therethrough.

Another aspect of the invention is a device useful for evaluating tumor cells *in vitro* (e.g., useful for evaluating metastasis of tumor cells *in vitro*, for evaluating tumor cell migration and/or invasion *in vitro*, for evaluating growth of a construct comprising tumor cells *in vitro*, and/or for evaluating response to a compound of interest, such as, e.g., a drug or prodrug), the device comprising: two or more chambers with at least one organoid in each chamber. The one or more organoids present in the device may be prepared from cells obtained from a single subject. In some embodiments, the device comprises a live tumor cell organoid in a first chamber and a liver organoid in a second chamber. The live tumor cell organoid and liver organoid may be formed from cells obtained from the same subject, which may provide a personalized analysis.

The foregoing and other objects and aspects of the invention are explained in greater detail below. It should be appreciated that the invention can be embodied in different forms and should not be construed as limited to the embodiments set forth herein. Rather, these embodiments are provided so that this disclosure will be thorough and complete, and will fully convey the scope of the invention to those skilled in the art.

### Brief Description of the Drawings

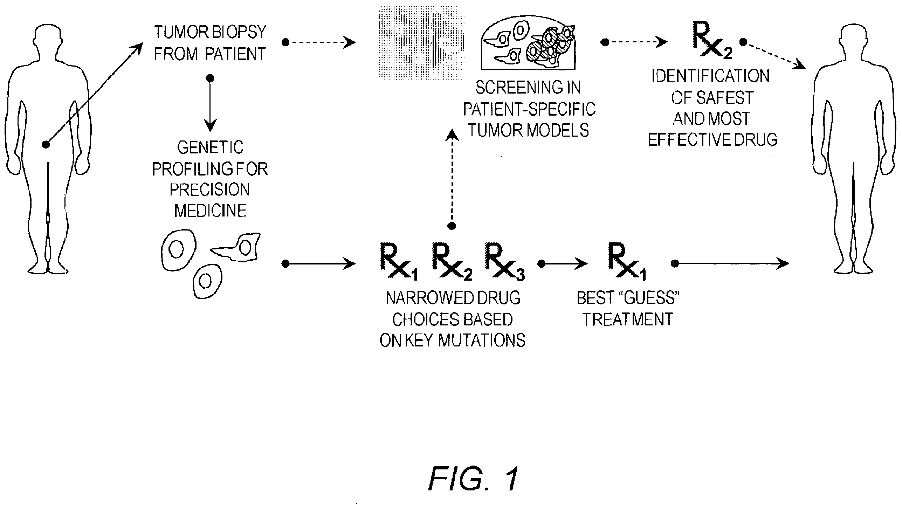

**Fig. 1** shows a concept of using engineered 3D in vitro models to better inform precision medicine treatment regimens on a patient-by-patient basis. Solid arrows: The current state of the art precision medicine pipeline, in which treatments are identified for patients based on their tumor genetic profiles. However, in practice, even after identification of key mutations, oncologists are often left with several potential drug options, resulting in a best guess or estimate for what the optimal treatment is. Dashed arrows: Implementation of organoids created with patient cells can supplement genetic screens of biopsied tumor cells, ultimately predicting the optimal therapies for patients.

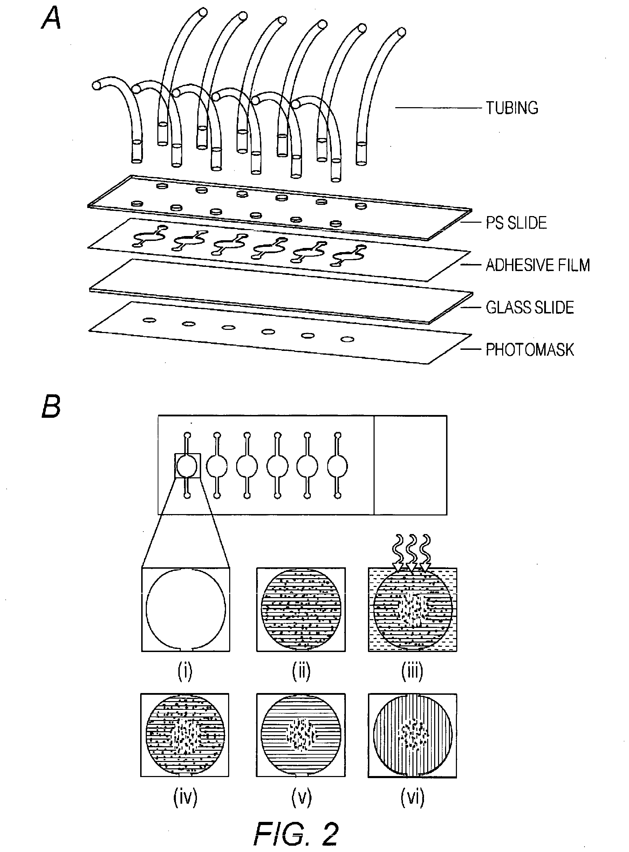

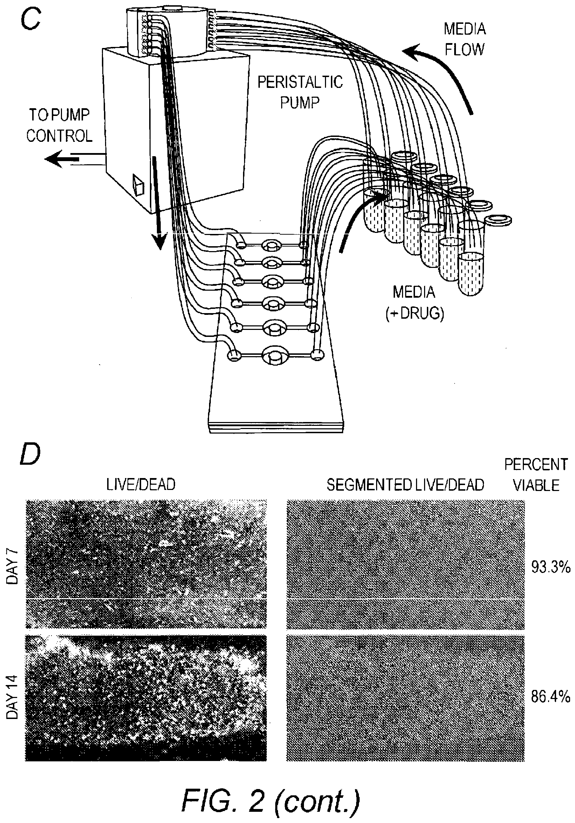

**Fig. 2** shows panel A) Schematic assembling of microfluidic device layers, which are from top to bottom: tubing, a polystyrene (PS) slide with fluid inlets and outlets, an adhesive film with chambers for an organoid and with corresponding fluid inlets and outlets, a glass slide, and a photomask layer; panel B) In situ patterning technique: a microfluidic chamber (i) is filled with hydrogel mixture containing HA hydrogel, photoinitiator and tumor cells (ii) and then exposed with UV ilumination through a photomask (iii). Exposed hydrogel is crosslinked (iv) after clean buffer is used to flush non-crosslinked gel (v). Flushing buffer is finally replaced with media (vi) for incubation; panel C) The total measurement set-up, featuring a low-volume, closed loop fluidic circuit for each organoid facilitated by a computer-controlled peristaltic pump; and panel D) Maximum projection confocal images and segmented images of tumor construct after LIVE/DEAD assays on day 7 and day 14 onboard showing percent viabilities of 93.3% and 86.4%, respectively.

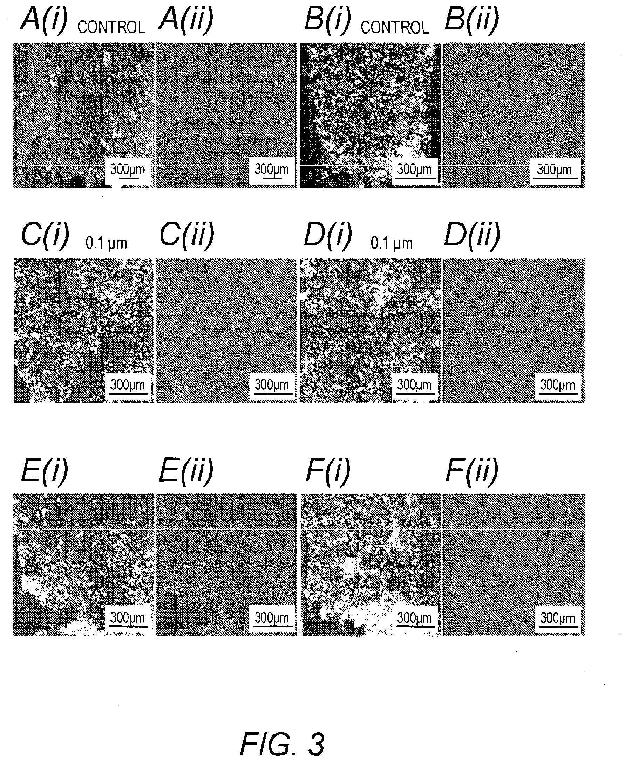

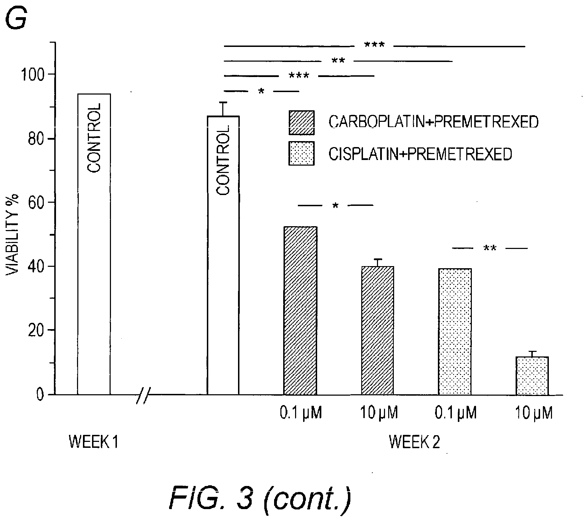

**Fig. 3** shows panels a-f(i), which are the maximum projection confocal images of tumor construct after LIVE/DEAD assay, and panels a-f(ii), which are segmented images using Imaris software. Medium grey represents dead cells and light grey represents live cells. Panel a) is week 1 control image with >90% viability, panel b) is week 2 control, panels c & d) show the construct under the influence of carboplatin and premetrexed drug for 7 days; and panels e & f) show the construct under the influence of cisplatin and premetrexed drug for 7 days. Panel g) shows a graph of viability (live cells/dead cells) measurements derived from segmented images for control and drug treated organoids. Drug exposure was initiated on day 7. Significance: * p<0.1, ** p<0.05, *** p<0.01.

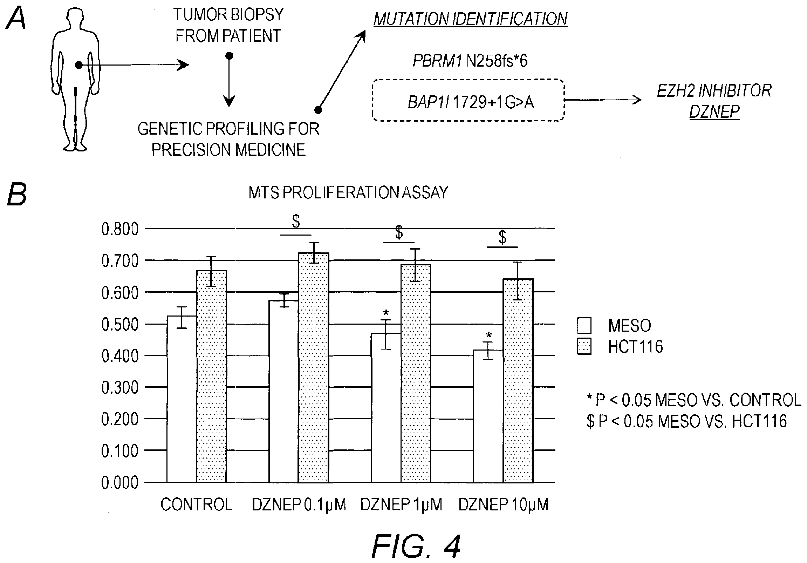

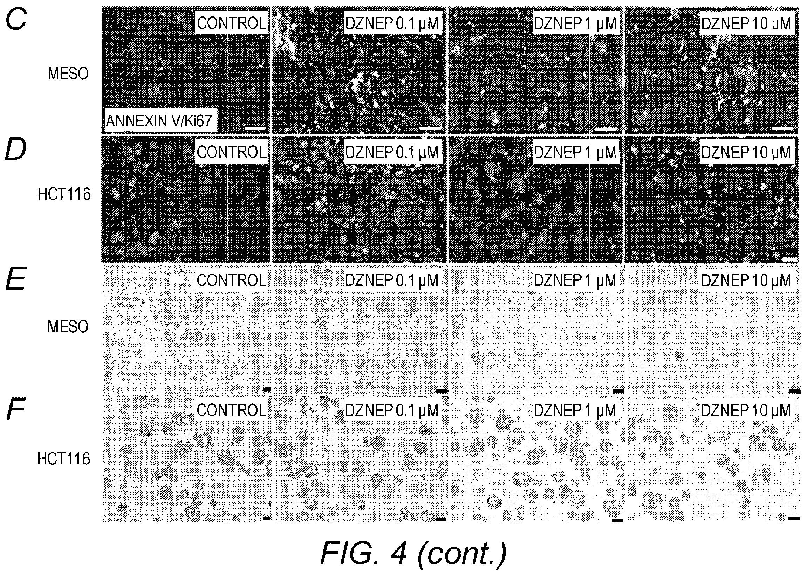

**Fig. 4** shows biomarker-driven therapeutic agent screening in patient-derived tumor constructs. Panel A) Genetic analysis of the tumor biopsy specimen identified two mutations, PBRM1 and BAP1. BAP1 mutations were identified as targetable by the EZH2 inhibitor DZNep. Panel B) MTS mitrochondrial metabolism assay shows control organoids were most proliferative in comparison to DZNep drug treatments which resulted in decreased mitochondrial metabolism, proportional to decreased cell number. Conversely, HCT116 organoids do not appear to respond negatively to DZNep. Panels C-D) Additionally, using Annexin V and Ki67 biomarker images, analysis was done showing that all drug treatments had higher ratios of Annexin V to Ki67 indicating a greater amount of apoptotic cells. Representative images of Annexin V and Ki67 fluorescence biomarkers are shown for DZNep treatments. Red stain (medium grey in black and white images) - annexin V; Green stain (light grey in black and white images) - Ki67. Scale bars indicate 100um. Panels E-F) Hematoxylin and eosin stained organoid sections show loss of normal morphology and appearance of ghost cells with no nuclei as the DZNep concentration increases in the mesothelioma organoids. Conversely, HCT116 cells do not show this dose dependent change, suggesting DZNep more effectively induces cell death in the mesothelioma organoids than the HCT116 organoids.

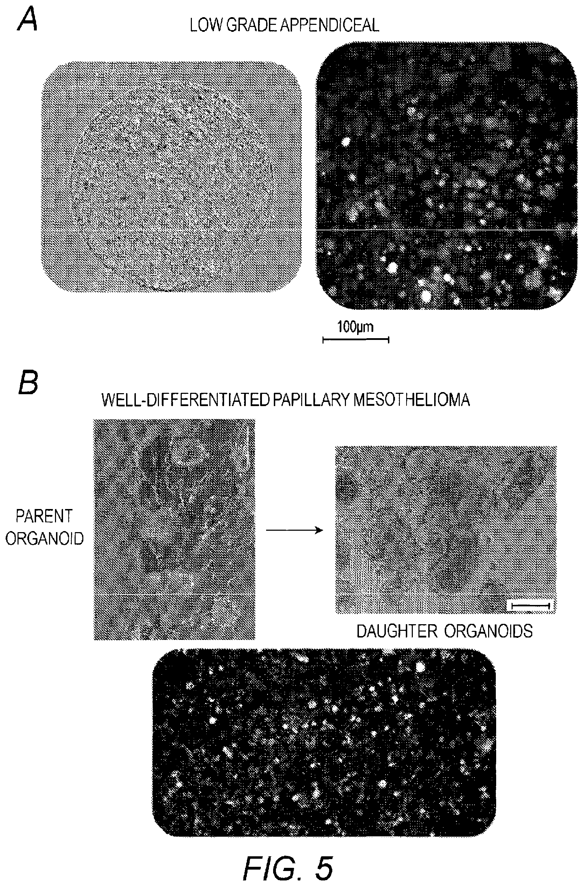

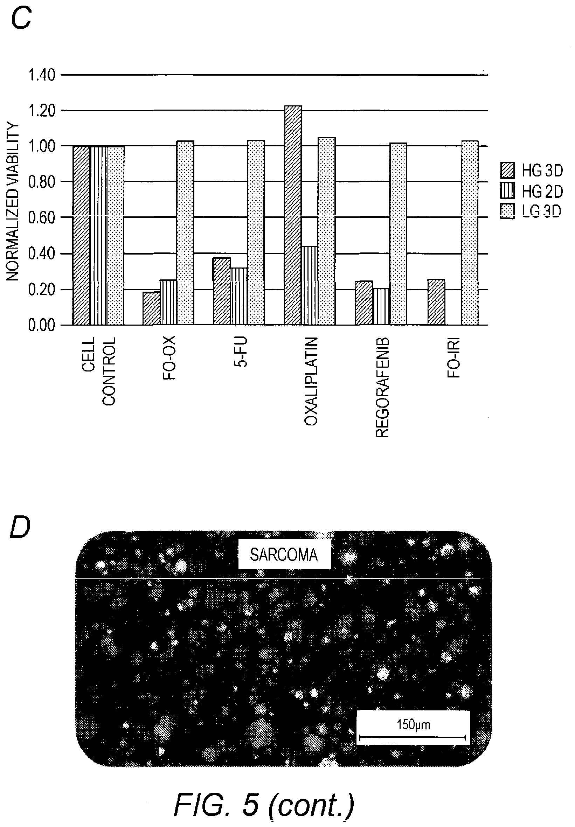

**Fig. 5** shows viable organoids from difficult to culture. Panel A) low grade appendiceal (LGA) and panel B) well-differentiated peritoneal papillary mesothelioma. Panel C) LGA organoids do not respond to therapies, as already suspected in clinical practice, whereas high grade appendiceal tumor organoids do. Panel D) Establishment of viable tumor organoids from a rare sarcoma.

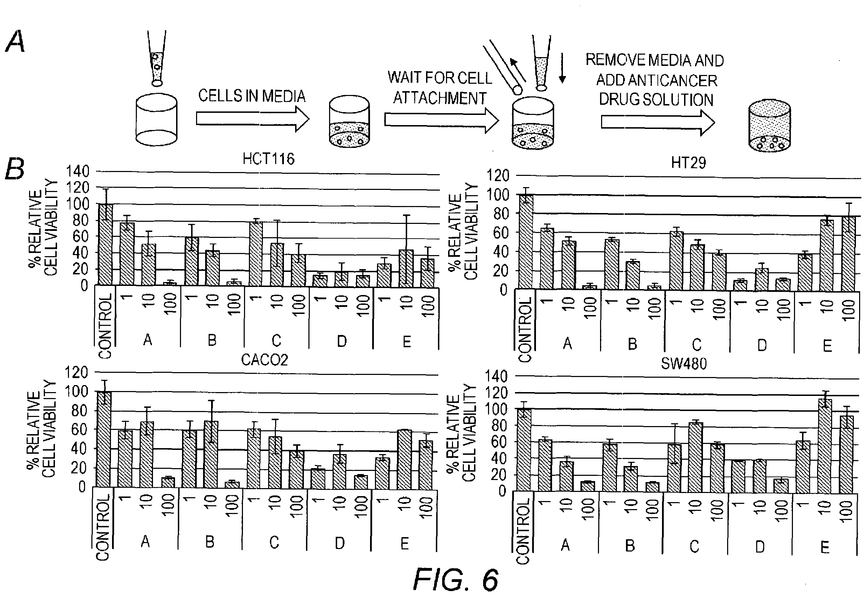

**Fig. 6** shows an illustration of the 2D Drug Study Protocol. Panel (A). Percent relative cell viability values of HCT116, HT29, CACO2, and SW480 colorectal cancer cell lines after being treated with various concentrations of anticancer drugs in a two-dimensional microenvironment. Panel (B) A= Regorafenib (µM) B= Sorafenib (µM) C= Trametinib (nM), D= 5-FU (mM) E= Drabrafenib (µM).

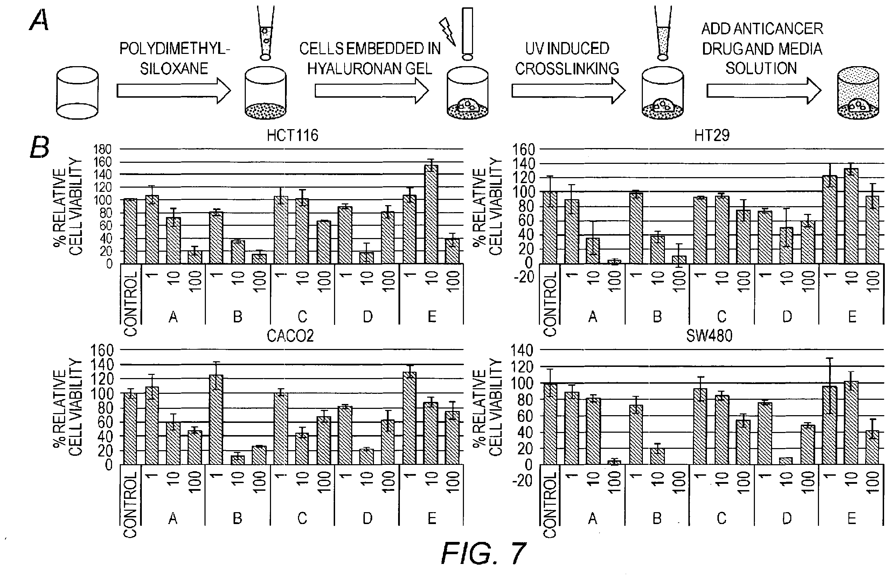

**Fig. 7** shows an illustration of the 3D Drug Study Protocol. Panel (A). Percent relative cell viability values of HCT116, HT29, CACO2, and SW480 colorectal cancer cell lines after being treated with various concentrations of anticancer drugs in a three-dimensional microenvironment. Panel (B) A= Regorafenib (µM) B= Sorafenib (µM) C= Trametinib (nM) D= 5-FU (mM) E= Drabrafenib (µM).

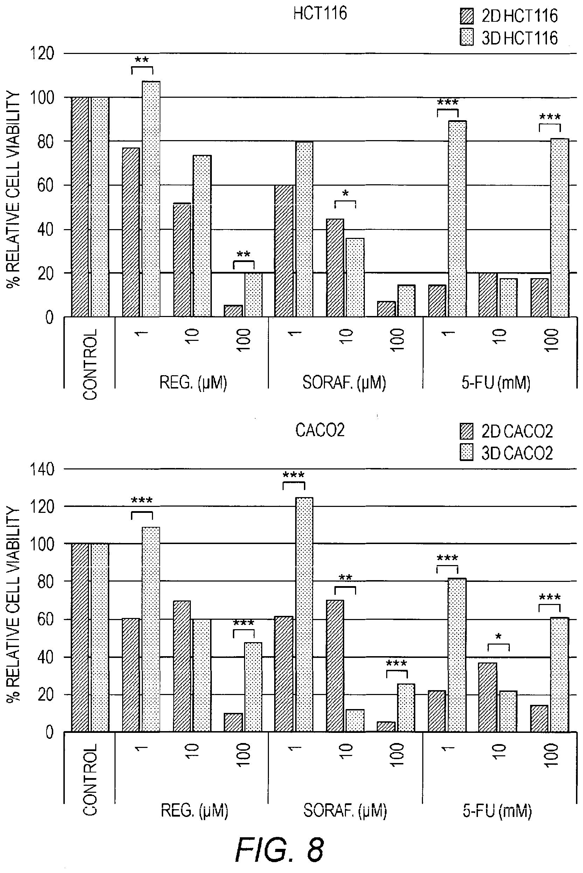

**Fig. 8** shows graphs comparing the results of the 2D and 3D drug screenings with Regorafenib, Sorafenib, and 5-FU on HCT116 and CACO2 colorectal cell lines. Significant difference as determined by p-values also labelled (< 0.10 = * < 0.05 = ** < 0.01 = ***).

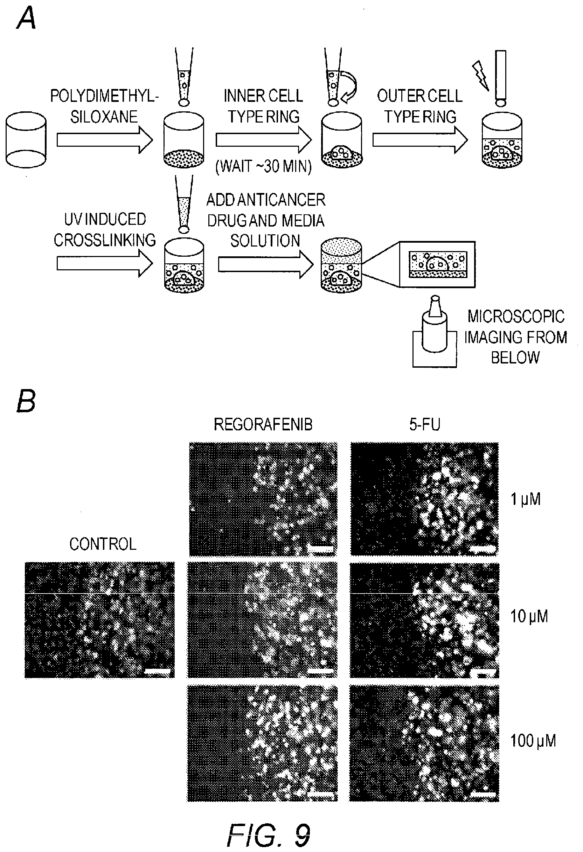

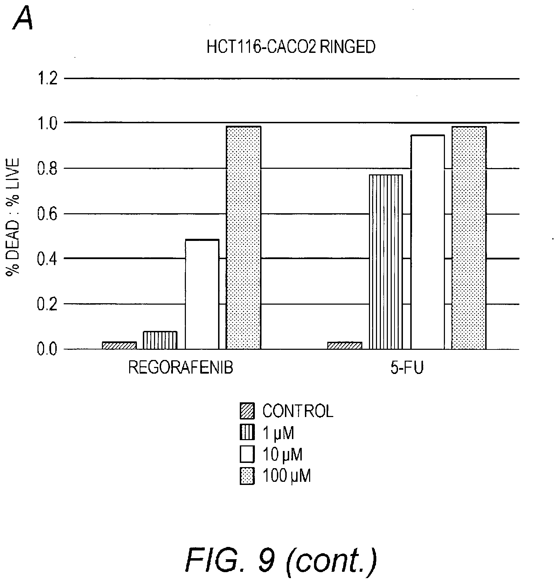

**Fig. 9** shows an illustration of the Ringed Organoid Drug Study Protocol. Panel (A). Microscopy images on Day 7 detailing the targeted cell death of HCT116 by Regorafenib and the universal cell death of both HCT116 and CACO2 by 5-FU. Panel (B) Green (light grey in black and white images) fluorescent dye (Calcein AM) illustrates live cells whereas red (medium grey in black and white images) fluorescent dye (Ethidium Homodimer-1) illustrates dead cells (scale bar = 200 µm). Panel (C) shows graphs of the ratios for each of the concentrations of Regorafenib and 5-FU and against the control, untreated ratio.

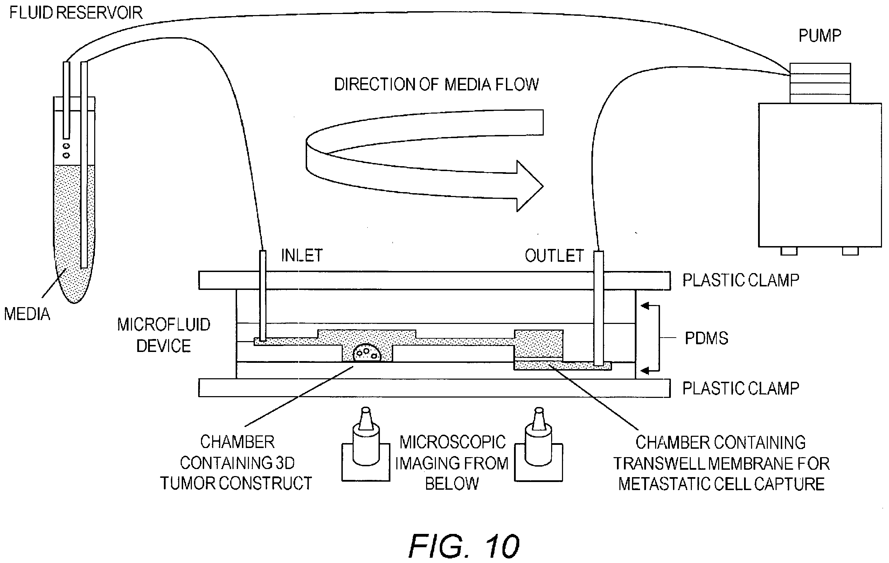

**Fig. 10** is an illustration depicting the interior components of the microfluidic device and the exterior components to circulate media through the device.

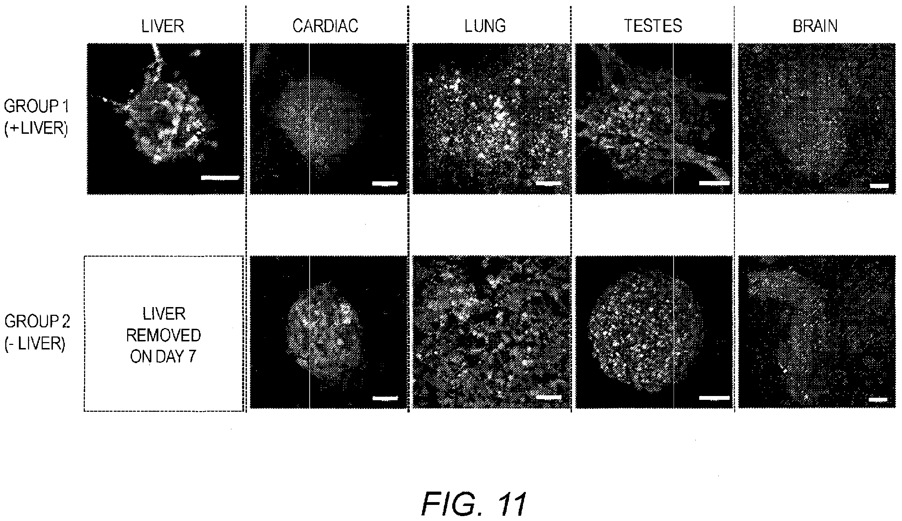

**Fig. 11** shows images of LIVE/DEAD results, which show that liver organoids are required to metabolize capecitabine to the active 5-FU form of the drug, inducing increased cell death in cardiac and lung organoids.

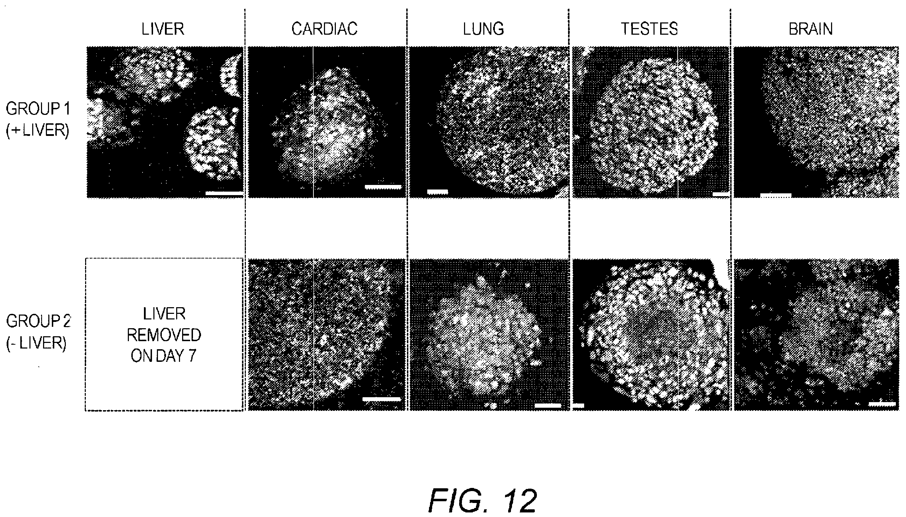

**Fig. 12** shows images of LIVE/DEAD results in the miniaturized system and show that, with liver present, capecitabine is metabolized into the toxic drug 5-FU, causing heart and lung toxicity. Without liver, this metabolism does not occur, and cardiac and lung organoid viability does not decrease.

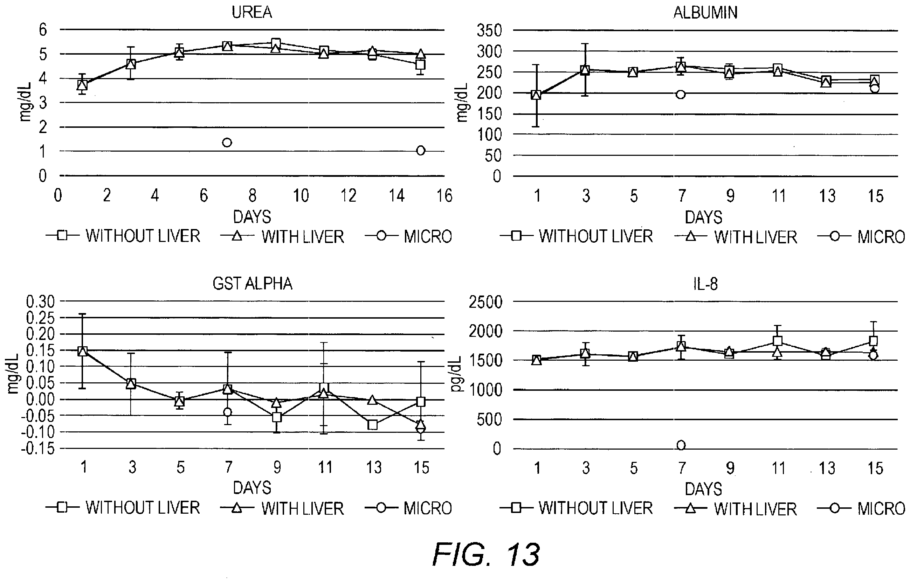

**Fig. 13** shows graphs of the initial biomarker analysis in the standard size system and the miniaturized system.

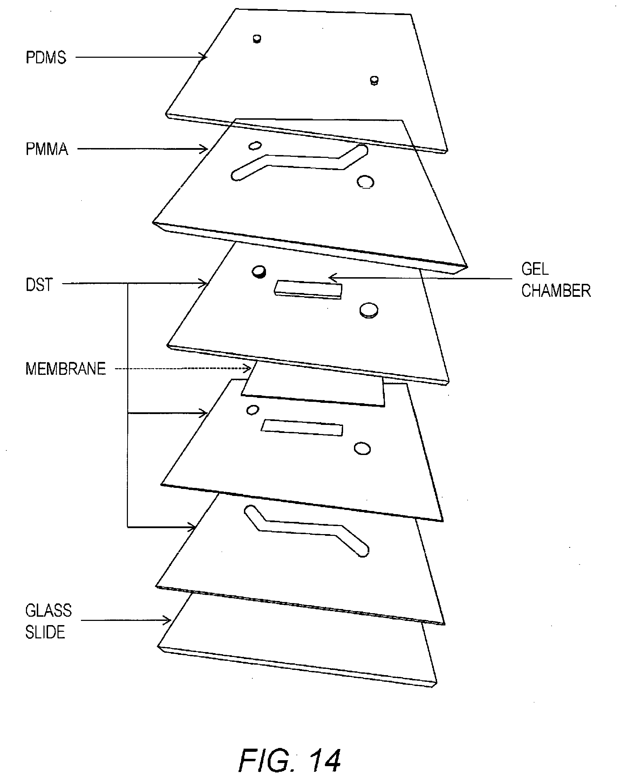

**Fig. 14** shows an illustration of the hybrid microreactor fabrication strategy incorporating tape fluidics and adhesive films and/or double sided tape (DST), poly(methyl methacrylate) (PMMA), and polydimethylsiloxane (PDMS) components.

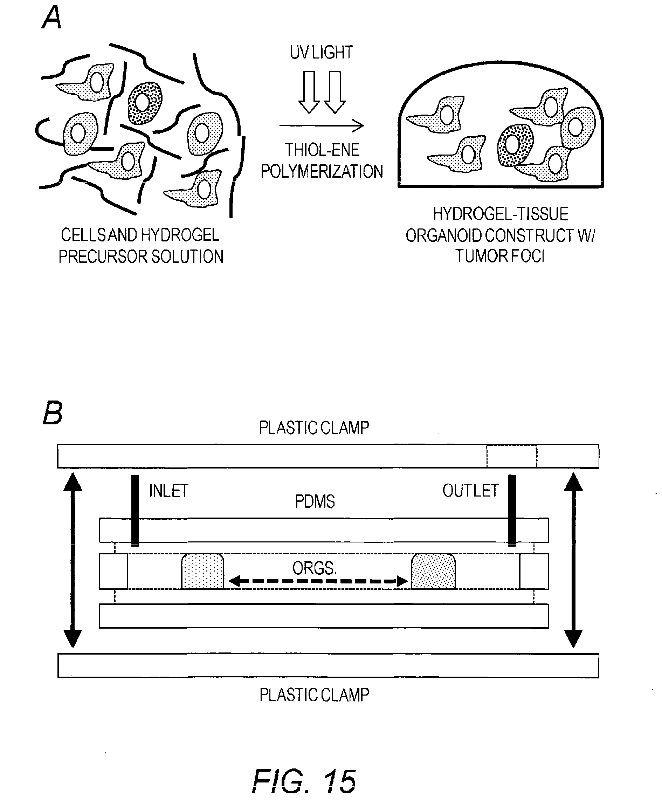

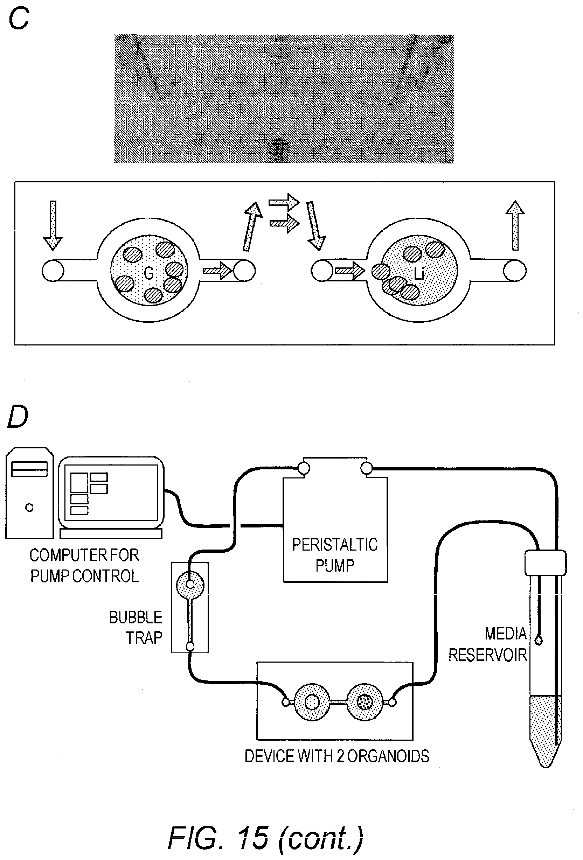

**Fig. 15** shows different components and/or views (panels A-D) of an example device having two chambers according to aspects of the present invention.

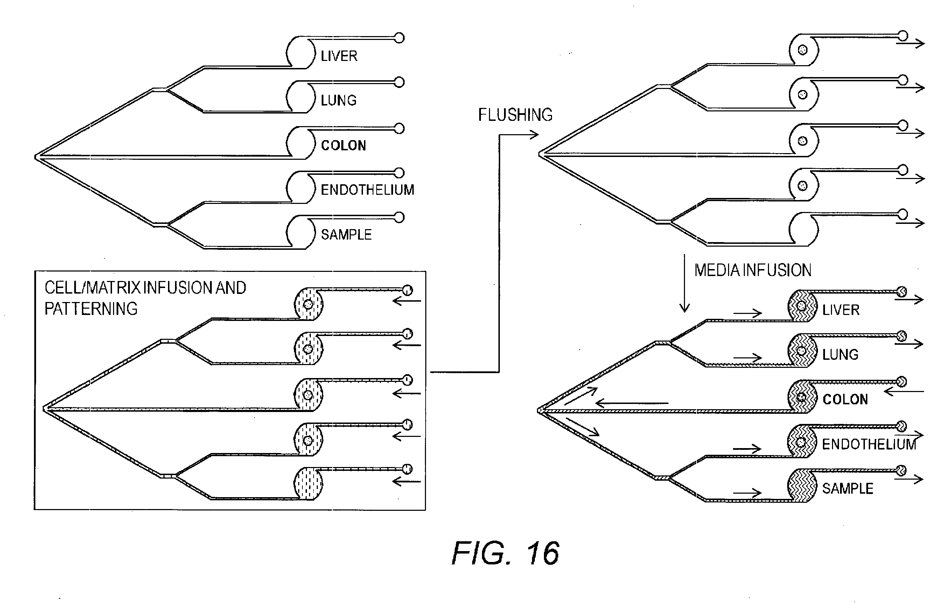

**Fig. 16** shows an example device having six chambers according to aspects of the present invention.

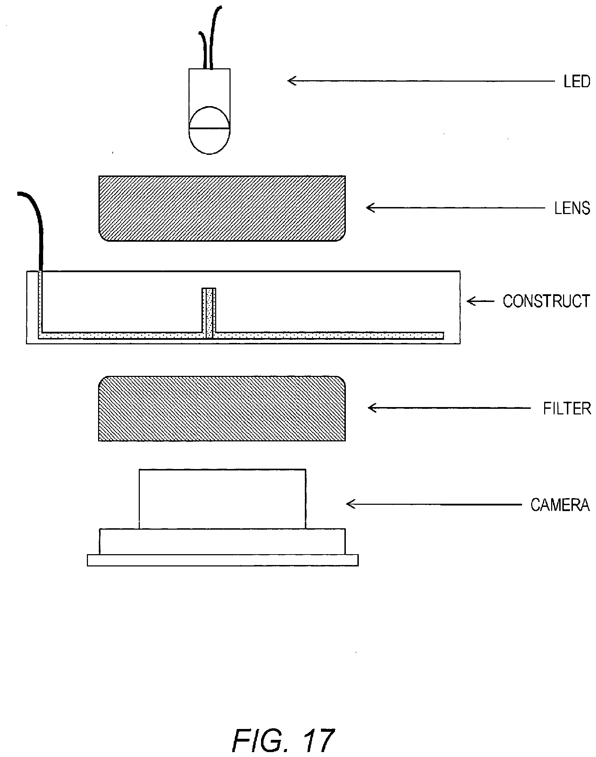

**Fig. 17** is a schematic of a device configuration according to aspects to of the present invention.

### Detailed Description of Illustrative Embodiments

The present invention is now described more fully hereinafter with reference to the accompanying drawings, in which embodiments of the invention are shown. This invention may, however, be embodied in many different forms and should not be construed as limited to the embodiments set forth herein; rather these embodiments are provided so that this disclosure will be thorough and complete and will fully convey the scope of the invention to those skilled in the art.

The terminology used herein is for the purpose of describing particular embodiments only and is not intended to be limiting of the invention. As used herein, the singular forms "a," "an" and "the" are intended to include plural forms as well, unless the context clearly indicates otherwise. It will be further understood that the terms "comprises" or "comprising," when used in this specification, specify the presence of stated features, integers, steps, operations, elements components and/or groups or combinations thereof, but do not preclude the presence or addition of one or more other features, integers, steps, operations, elements, components and/or groups or combinations thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity. All publications, patent applications, patents and other references mentioned herein are incorporated by reference in their entirety. In case of a conflict in terminology, the present specification is controlling.

Also as used herein, "and/or" refers to and encompasses any and all possible combinations of one or more of the associated listed items, as well as the lack of combinations when interpreted in the alternative ("or").

Unless the context indicates otherwise, it is specifically intended that the various features of the invention described herein can be used in any combination. Moreover, the present invention also contemplates that in some embodiments of the invention, any feature or combination of features set forth herein can be excluded or omitted. To illustrate, if the specification states that a complex comprises components A, B and C, it is specifically intended that any of A, B or C, or a combination thereof, can be omitted and disclaimed.

As used herein, the transitional phrase "consisting essentially of" (and grammatical variants) is to be interpreted as encompassing the recited materials or steps "and those that do not materially affect the basic and novel characteristic(s)" of the claimed invention. Thus, the term "consisting essentially of" as used herein should not be interpreted as equivalent to "comprising."

The term "about," as used herein when referring to a measurable value, such as an amount or concentration and the like, is meant to refer to variations of up to ± 20% of the specified value, such as, but not limited to, ± 10%, ± 5%, ± 1%, ± 0.5%, or even ± 0.1% of the specified value, as well as the specified value. For example, "about X" where X is the measurable value, is meant to include X as well as variations of ± 20%, ± 10%, ± 5%, ± 1%, ± 0.5%, or even ± 0.1% of X. A range provided herein for a measureable value may include any other range and/or individual value therein.

"Cells" used in the present invention are, in general, animal cells, particularly mammalian and primate cells, examples of which include but are not limited to human, dog, cat, rabbit, monkey, chimpanzee, cow, pig, or goat. The cells are preferably differentiated at least in part to a particular cell or tissue type, such as liver, intestine, pancreas, lymph node, smooth muscle, skeletal muscle, central nerve, peripheral nerve, skin, immune system, etc. Some cells may be cancer cells, as discussed further below, in which case they optionally but preferably express (naturally, or by recombinant techniques) a detectable compound, as also discussed further below.

"Subjects" as used herein are, in general, human subjects, although aspects of the invention may be implemented with other animal subjects, particularly mammalian subjects (e.g., dogs, cats, horses, goats, sheep) for veterinary purposes. Subjects may be male or female and of any age, including infant, juvenile, adolescent, adult, and geriatric.

"Organoid" is used interchangeably with "cell construct" and "three dimensional tissue construct" herein, and refers to a composition of live cells, typically in a carrier media, arranged in a three-dimensional or multi-layered configuration (as opposed to a monolayer). Suitable carrier media include hydrogels, such as cross-linked hydrogels as described below. Additional example hydrogels include, but are not limited to, those described in PCT/US2015/055699, the contents of which are incorporated herein by reference in their entirety. Organoids may comprise one differentiated cell type, or two or more differentiated cell types, depending upon the particular tissue or organ being modeled or emulated. Some organoids may comprise cancer cells, as discussed further below. Where organoids comprise cancer cells, they may include tissue cells, and/or may include a tissue mimic without cells, such as an extracellular matrix (or proteins or polymers derived therefrom), hyaluronic acid, gelatin, collagen, alginate, etc., including combinations thereof. Thus, in some embodiments, cells are mixed together with the extracellular matrix, or cross-linked matrix, to form the organoid or construct, while in other embodiments cell aggregates such as spheroids or organoids may be pre-formed and then combined with the extracellular matrix. In some embodiments, the organoid is a neoplastic organoid and/or a non-neoplastic organoid.

In some embodiments, the organoid comprises cells that are human-derived cells, and, in some embodiments, the organoid comprises cells that consist of human-derived cells. An organoid of the present invention may express and/or produce one or more biomarkers (e.g., 1, 2, 3, 4, or more) that are the same as a biomarker produced by the cells *in vivo.* For example, liver cells *in vivo* produce albumin and an organoid of the present invention comprising liver cells may express albumin. In some embodiments, an organoid may express a biomarker in the same amount or in an amount that is ±20%, ±10%, or ±5% of the average amount produced and/or expressed by corresponding cells *in vivo.* Example biomarkers include, but are not limited to, albumin, urea, glutathione S-transferase (GST) (e.g., α-GST), chemokines (e.g., IL-8, IL-1β, etc.), prostacyclin, SB100B, neuron-specific enolase (NSE), myelin basic protein (MBP), hormones (e.g., testosterone, estradiol, progesterone, etc.), inhibin A/B, lactate dehydrogenase (LDH), and/or tumor necrosis factor (TNF).

In some embodiments, an organoid of the present invention is about 200 µm to about 350 µm in diameter, such as, for example, about 200,250, 300, or 350 µm. The organoid may comprise about 1,500 or 2,000 to about 3,000 or 3,500 cells in total. An organoid of the present invention may be in any suitable shape, such as, e.g., any three-dimensional shape or multi-layered shape. In some embodiments, an organoid of the present invention is in the form of a spheroid. In some embodiments, an organoid of the present invention may be self-organized in a suspension or medium (e.g., a cross-linked hydrogel).

"Growth media" as used herein may be any natural or artificial growth media (typically an aqueous liquid) that sustains the cells used in carrying out the present invention. Examples include, but are not limited to, an essential media or minimal essential media (MEM), or variations thereof such as Eagle's minimal essential medium (EMEM) and Dulbecco's modified Eagle medium (DMEM), as well as blood, blood serum, blood plasma, lymph fluid, etc., including synthetic mimics thereof. In some embodiments, the growth media includes a pH color indicator (e.g., phenol red).

"Candidate compound" or "compound of interest" as used herein may be any compound for which activity in inhibiting the spreading of cancer cells from a primary site to a second site is to be determined, or for which activity in deceasing cancer cell viability and/or anti-tumor activity is to be determined. For demonstrative purposes, Marimastat (N-[2,2-dimethyl-1-(methylcarbamoyl)propyl]-2- [hydroxy-(hydroxycarbamoyl)methyl]-4-methyl-pentanamide) is used as a test compound. However, any compound may be used, typically organic compounds such as proteins, peptides, nucleic acids, and small organic compounds (aliphatic, aromatic, and mixed aliphatic/aromatic compounds) may be used. Candidate compounds may be generated by any suitable techniques, including randomly generated by combinatorial techniques, and/or rationally designed based on particular targets. *See, e.g.,* A.M. Stock et al., Targets for anti-metastatic drug development, Curr. Pharm. Des. 19(28): 5127-34 (2013). In some embodiments, the candidate compound is a prodrug, which may be converted to an active drug during testing and/or a method of the present invention. In some embodiments, a prodrug is converted to an active drug by a liver organoid.

"Detectable compound" as used herein may be a fluorescent protein (*e.g.*, red fluorescent protein, green fluorescent protein, etc.), an antigenic protein or peptide to which an antibody coupled to an enzyme, fluorescent, or radioactive group, or other label, will specifically bind), or any other suitable detectable compound. The detectable compound may be one naturally occurring in a cancer cell (*e.g.*, a cell marker protein that is expressed at higher levels in cancer cells than non-cancer cells), or one inserted into cancer cells by genetic engineering/recombinant DNA techniques (i.e., heterologous).

Cells used to carry out the present invention are preferably animal cells (*e.g.*, bird, reptile, amphibian, etc.) and in some embodiments are preferably mammalian cells (*e.g.*, dog, cat, mouse, rat, monkey, ape, human). The cells may be differentiated or undifferentiated cells, but are in some embodiments tissue cells (*e.g.*, liver cells such as hepatocytes, pancreatic cells, cardiac muscle cells, skeletal muscle cells, etc.).

Choice of cells will depend upon the particular organoid being created. For example, for a liver organoid, liver hepatocyte cells may be used. For a peripheral or central nerve organoid, peripheral nerve cells, central nerve cells, glia cells, or combinations thereof may be used. For a bone organoid, bone osteoblast cells, bone osteoclast cells, or combinations thereof may be used. For a lung organoid, lung airway epithelial cells may be used. For a lymph node organoid, follicular dendritic lymph cells, fibroblastic reticular lymph cells, leucocytes, B cells, T cells, or combinations thereof may be used. For a smooth or skeletal muscle organoid, smooth muscle cells, skeletal muscle cells, or combinations thereof may be used. For a skin organoid, skin keratinocytes, skin melanocytes, or combinations thereof may be used. The cells may be differentiated upon initial incorporation into the composition, or undifferentiated cells that are subsequently differentiated may be used. Additional cells may be added to any of the compositions described above, and cancer cells as described below may be added to primary or "first" organoids, as described below.

Cancer cells used in the present invention may be any type of cancer cell, including but not limited to melanoma, carcinoma, sarcoma, blastoma, glioma, and astrocytoma cells, etc. In some embodiments, the cancer cells used in the present invention express N-cadherin in the methods taught herein, and/or show epithelial to mesenchymal transition. Cancer cells may be cancer cells from any tissue of origin, including but not limited to intestinal (small intestine, large intestine, colon), lung, breast, prostate, skin, bone, brain, liver, pancreatic, uterine, cervical, testicular, and ovarian cancer cells, etc.

In some embodiments, cells may be obtained from a subject, such as, for example, a subject or patient undergoing treatment for cancer and/or that has cancer and/or a subject that has a compromised immune system. In some embodiments, cells are tumor cells, such as, e.g., biopsy-derived tumor cells, and organoids prepared from such cells may be used to screen potentially effective drugs and/or treatments. Example biopsy-derived tumor organoids include, but are not limited to, mesothelioma, colorectal, appendiceal, lung, melanoma, and sarcoma organoids. In some embodiments, the cells include benign cells obtained from a tissue biopsy. The cells may be differentiated at least in part to a particular cell or tissue type, such as brain, liver, intestine, pancreas, lymph node, smooth muscle, skeletal muscle, central nerve, peripheral nerve, skin, immune system, etc. Biopsy-derived cells (e.g., tumor and/or benign) may be used to form and/or prepare an organoid of the present invention, and the resulting organoid may be prepared and/or used in a method and/or device of the present invention within about 1, 2, 3, 4, 5, 6, 7, or 8 days after the biopsy. In some embodiments, the cells may be labeled with a detectable compound, such as, but not limited to, with a fluorescent compound (e.g., dye, protein, etc.).

In some embodiments, an organoid of the present invention is not prepared from and/or does not comprise cells from an immortalized cell line. Organoids of the present invention may comprise and/or be prepared using tumor cells and/or high functioning cells, such as, but not limited to, primary cells and/or stem cells, e.g., induced pluripotent stems and/or differentiated iPS-derived cells.

Aspects and features of the inventions described herein can be implemented in accordance with known materials, methods and techniques, or variations thereof that will be apparent to those skilled in the arts to which the present inventions pertain. *See, e.g.*, Skardal A, Devarasetty M, et al. A reductionist metastasis-on-a-chip platform for in vitro tumor progression modeling and drug screening. Biotechnology and Bioengineering 113(9), 2020-32 (2016); Skardal A, Devarasetty M, et al. A hydrogel bioink toolkit for mimicking native tissue biochemical and mechanical properties in bioprinted tissue constructs. Acta Biomaterialia 25, 24-34 (2015); Bhise N, Manoharan V, et al. A liver-on-a-chip platform with bioprinted hepatic spheroids. Biofabrication 8(1); 014010 (2016).

As noted above, the present invention provides an *in vitro* cell construct (or "organoid") useful as a tumor model, comprising: *(a)* a core comprised of live tumor cells; and *(b)* a shell surrounding (*e.g.*, encapsulating) the core, the shell comprised of live benign cells (*e.g.*, tissue cells, benign or differentiated tumor cells, etc.).

In some embodiments, the live tumor cells comprise malignant cells.

In some embodiments, the live tumor cells comprise or consist of colorectal cancer cells (*e.g.*, HCT116 cells, HT29 cells, SW480 cells, Caco2 cells, etc.).

In some embodiments, the tumor cells and the benign cells comprise mammalian cells (*e.g.*, human, mouse, rat, monkey, etc.).

In some embodiments, the live benign cells comprise epithelial cells.

In some embodiments, the live benign cells comprise Caco-2 cells.

In some embodiments, the tumor cells contain a detectable compound (e.g., a fluorescent compound).

In some embodiments, the core and/or the shell comprise a hydrogel.

In some embodiments, the constructs are grown in culture for a time of 1 or 2 days, or 1 to 2 weeks, or more.

Provided according to some embodiments is an *in vitro* cell construct (or "organoid") useful as a tumor model that comprises live tumor cells from a subject. In some embodiments, the live tumor cells are obtained and/or derived from the subject, such as, e.g., a tumor in the subject. The live tumor cells may be obtained and/or derived from a tumor biopsy. In some embodiments, the live tumor cells form the core of the organoid and the organoid comprises a shell of live benign cells.

In some embodiments, an organoid is provided that comprises live benign cells from a subject. The benign cells may be obtained and/or derived from the subject, such as, e.g., a tissue in the subject. In some embodiments, the live benign cells may be obtained and/or derived from a tissue biopsy and/or may be tissue specific. An organoid comprising live benign cells may be separate (e.g., separately formed and/or present in a different chamber of a device) from an organoid comprising live tumor cells. In some embodiments, at least two (e.g., 2, 3, 4, 5, 6, 7, 8, or more) different organoids are formed with cells obtained and/or derived from a single subject (e.g., using one or more biopsies from the subject), with at least one organoid comprising live tumor cells from a tumor biopsy from the subject and at least one separate organoid comprising live liver cells from the subject. In some embodiments, at least two (e.g., 2, 3, 4, 5, 6, 7, 8, or more) organoids are formed with cells obtained and/or derived from a single subject (e.g., using one or more biopsies from the subject), with at least one organoid comprising live tumor cells from a tumor biopsy from the subject and at least one separate organoid comprising live benign cells from the subject that are the same tissue type as the liver tumor cells.

An organoid of the present invention may provide a patient-specific model system, which may be used to determine a treatment for the patient, optionally before initiation of therapy and/or treatment. In some embodiments, a compound of interest may be screened for anti-tumor activity, optionally in addition to an additional screening method, such as, but not limited to, genetic biomarker assessment and/or genetic profiling. In some embodiments, an organoid and/or method of the present invention may allow and/or provide for cellular biomarker recognition, biomarker expression quantification, and/or real time testing of chemotherapy drug efficacy.

A method of screening a compound of interest *in vitro* for anti-tumor activity can be carried out by: *(a)* providing at least one construct comprising live tumor cells as described herein; *(b)* contacting the compound to the construct *in vitro*; and then *(c)* determining the growth of the live tumor cells (*e.g.*, as compared to tumor cells of at least one like construct not contacted the compound, and/or as compared to live benign cells present in a shell around the construct and/or present in a separate organoid), a decrease in growth of the live tumor cells (*e.g.*, lack of proliferation of the tumor cells, death of the tumor cells, decrease in invasion of the shell by the tumor cells, etc.), indicating anti-tumor activity of the compound of interest. In some embodiments, the tumor cells are collected from a patient afflicted with the tumor. In some embodiments, the compound of interest is a prodrug. In some embodiments, the method may further comprise administering the compound to the patient in a treatment-effective amount when the compound is determined to decrease the growth of the tumor cells *in vitro.*

In some embodiments, a method of the present invention comprises obtaining a tumor biopsy from a subject (e.g., a patient) and using the cells to prepare an organoid of the present invention. The tumor biopsy may be genetically sequenced in part or in full in order to identify mutations, and any mutations identified may indicate and/or suggest one or more compound(s) of interest for therapeutic purposes for the subject (e.g., anti-tumor activity). The method may comprise screening the one or more compound(s) of interest that were identified in the genetic sequencing and contacting each of the one or more compound(s) of interest and/or a combination thereof to the organoid prepared using the cells from the tumor biopsy. The method may further comprise administering one or more of the compound(s) of interest to the subject from which tumor biopsy was taken in a treatment-effective amount when the one or more compound(s) is determined to decrease the growth of the tumor cells *in vitro.*

In some embodiments, a method of the present invention comprises preparing a tumor cell organoid from live tumor cells obtained from a tumor biopsy from a subject and preparing a liver organoid from live liver cells obtained from the same subject or a different source. In some embodiments, the live tumor cells and live liver cells are obtained from the same subject. The live tumor cell organoid and liver organoid may be provided in fluid communication with one another (e.g., in a device, such as, e.g., a device as described herein), and a compound of interest may be contacted to the live tumor cell organoid and/or liver organoid. Further, the growth of the live tumor cells in the live tumor cell organoid may be determined. In some embodiments, the compound of interest is a prodrug, which the liver organoid may metabolize to provide an active drug, and the activity of the active drug may be determined (e.g., it may be determined if the growth of the live tumor cells and/or liver cells is affected by the prodrug and/or active drug). One or more additional organoids may be used in a method of the present invention. In some embodiments, the one or more additional organoids may be derived from the same subject and may be from the same tissue or a different tissue in the subject.

In some embodiments, a method and/or device of the present invention may provide and/or allow for parallel screening of two of more (e.g., 2, 3, 4, 5, 6, 7 or more) compounds of interest. In some embodiments, at least one of the two or more compounds of interest is a prodrug.

In some embodiments, a method and/or device of the present may provide and/or allow for results (e.g., drug screening results and/or therapeutic analysis) within about 1 or 2 weeks (e.g., within about 2, 3, 4, 5, 6, 7, 8, 9, or 10 days) after a biopsy (e.g., a tumor biopsy) from a subject is obtained, the cells of which were used to prepare an organoid of the present invention that was used in the method and/or device.

Also described herein is a device useful for evaluating tumor cells *in vitro*, including: *(a)* a microfluidic device having a chamber, and a channel in fluid communication with the chamber; *(b)* a live tumor cell construct (*e.g.*, an organoid) in the chamber; *(c)* a growth media in the chamber and the channel; *(d)* a pump operatively associated with the chamber and channel and configured for circulating the media from the chamber through the channel and back to the chamber; and *(e)* a microporous membrane (*e.g.*, a TRANSWELL^{®} microporous membrane) in the channel and positioned so that the media flows therethrough. In some embodiments, the device is useful for evaluating metastasis of tumor cells *in vitro*, for evaluating tumor cell migration and/or invasion *in vitro*, for evaluating growth of a construct comprising tumor cells *in vitro*, and/or for evaluating response to a compound of interest. In some embodiments, the device may be useful for evaluating construct size *in vitro*, the number of tumor cells *in vitro* and/or tumor cell death *in vitro.*

In some embodiments, a device useful for evaluating tumor cells *in vitro* comprises two or more chambers with at least one organoid in each chamber and being in fluid communication with one another. The device may be useful for evaluating metastasis of tumor cells *in vitro*, for evaluating tumor cell migration and/or invasion *in vitro*, for evaluating growth of a construct comprising tumor cells *in vitro*, and/or for evaluating response to a compound of interest. In some embodiments, the device may be useful for evaluating construct size *in vitro,* the number of tumor cells *in vitro* and/or tumor cell death *in vitro.* The one or more organoids present in the device may be prepared from cells obtained from a single subject. In some embodiments, the device comprises a live tumor cell organoid in a first chamber and a liver organoid in a second chamber. The live tumor cell organoid and liver organoid may be formed from cells obtained from the same subject, which may provide a personalized analysis.

In some embodiments, the device may comprise: a primary chamber comprising a live tumor cell organoid; at least one secondary chamber comprising a different organoid; at least one primary conduit connecting the primary and secondary chambers and providing fluid communication (e.g., such as the flow of a growth media) therebetween; and optionally a growth media in the primary chamber, each secondary chamber, and the primary conduit. In some embodiments, two or more (e.g., 2, 3, 4, 5, 6, 7, 8 or more) secondary chambers are provided with each comprising an organoid comprising different cells than another. In some embodiments, the at least one secondary chamber comprises a liver organoid, optionally wherein the live tumor cell organoid and liver organoid are prepared from cells obtained from the same subject. Additional example devices include, but are not limited to, those described in PCT/US2016/054611, the contents of which are incorporated herein in their entirety.

An example device comprising two chambers is shown in **Fig. 15**. Another example device having five champers is shown in **Fig. 16** along with how a medium may flow through the chambers of the device at different stages. **Fig. 17** shows a schematic of a device configuration, which may be used to track cells (e.g., tumor cells) labeled with a detectable compound according to aspects to of the present invention.

In some embodiments, the tumor cell construct comprises a hydrogel.

In some embodiments, the tumor cell constructs consist of a single cell type.

In some embodiments, the tumor cell constructs comprise or consist of colorectal cancer cells (*e.g.*, HCT116 cells, HT29 cells, SW480 cells, Caco2 cells, etc.).

In some embodiments, at least a portion of the microfluidic device is transparent, the device further comprising a detector (*e.g.*, a camera) operatively associated with the microporous membrane and configured for detecting (*e.g.*, imaging) tumor cells on the microporous membrane.

In some embodiments, a device used in a method of the present invention may be configured to provide a physiological or hyperphysiological fluid to tissue volume ratio. For example, one or more chambers of the device may have an average volume in a range of about 2 µL to about 10 µL. In some embodiments, one or more chambers of the device may have an average volume of about 2, 3, 4, 5, 6, 7, 8, 9, or 10 µL. In some embodiments, the device (e.g., an apparatus comprising 1, 2, 3, 4, 5, 6, or more chambers) may use liquid in the amount of and/or have a volume of less than about 100 µL, such as, e.g., about 95, 90, 85, 80, 75, 70, 65, 60, 55, 50, 45, 40 µL or less. The volume of the device, as used herein, refers to the volume to fill the chamber(s) and channel(s) of the device. In some embodiments, the device may use liquid in the amount of and/or have a volume of less than about 50 µL.

An organoid of the present invention may be viable for at least 1, 2, 3, 4, or more weeks. In some embodiments, an organoid in a device and/or as used in a method of the present invention may be viable for at least 1, 2, 3, 4, or more weeks. In some embodiments, an organoid of the present invention may be viable and may comprise at least about 75% or more (e.g., about 80%, 85%, 90%, 95% or more) living cells based on the average number of cells present in the organoid at 1, 2, 3, 4, or more weeks.

As described herein, cells and/or a cell sample may be used in a method of the present invention to form an organoid of the present invention. Methods of the present invention can provide a viable organoid. In some embodiments, a method of the present invention can achieve a take rate of at least 50% or more such as, e.g., about 50%, 55%, 60%, 65%, 70%, 75%, 80%, 85%, 90%, 95%, 98%, or more. For example, a 90% take rate means that 90% of the time a viable organoid or plurality of organoids (e.g., an organoid set) is achieved and/or provided by a method of the present invention. That is, for a 90% take rate, 9 out of 10 cell samples (e.g., tumor cell samples) yield a viable organoid or plurality of organoids when prepared according to a method of the present invention. The organoid or plurality of organoids may be used in another method of the present invention and/or diagnostics. In some embodiments, the cells and/or cell sample may be from a tumor, such as, e.g., a mesothelioma biospecimen and/or a GI tumor biospecimen.

A method of screening tumor cells *in vitro* for metastatic activity may be carried out by: *(a)* providing a device as described herein above; *(b)* circulating media in the device; and *(c)* quantitatively or qualitatively detecting tumor cells captured on the microporous membrane, where a greater number of tumor cells captured (*e.g.*, as compared to other tumor cells under like conditions, and/or non-metastatic cells under like conditions) indicates greater metastatic activity of the tumor cells.

A method of screening tumor cells *in vitro* for metastatic activity may be carried out by: *(a)* providing a device as described herein above (e.g., a device comprising a primary chamber comprising a live tumor cell organoid and at least one secondary chamber); *(b)* circulating media in the device; and *(c)* quantitatively or qualitatively detecting tumor cells present in one or more of the secondary chamber(s) (e.g., optionally on and/or in an organoid present in a secondary chamber), where a greater number of tumor cells present in one or more of the secondary chamber(s) (*e.g.*, as compared to other tumor cells under like conditions, and/or non-metastatic cells under like conditions) indicates greater metastatic activity of the tumor cells. A method of screening a compound of interest *in vitro* for anti-metastatic activity and/or anti-tumor activity may be carried out by: *(a)* providing a device as described herein above; *(b)* circulating media in the device; *(c)* administering the compound to the tumor cells (*e.g.*, by adding the compound to the media); and then *(d)* quantitatively or qualitatively detecting tumor cells captured on the microporous membrane, where a lesser number of tumor cells captured (*e.g.*, as compared to other tumor cells under like conditions but without the administering of the compound of interest) indicates greater anti-metastatic activity of the compound of interest. In some embodiments, the tumor cells are collected from a patient afflicted with the tumor. In some embodiment, the method further includes administering the compound to the patient in a treatment-effective amount when the compound is determined to decrease the metastasis of the tumor cells *in vitro*, decrease construct size *in vitro*, decrease the number of tumor cells *in vitro* and/or induce tumor cell death *in vitro.*

A method of screening a compound of interest *in vitro* for anti-metastatic activity and/or anti-tumor activity may be carried out by: *(a)* providing a device as described herein above; *(b)* circulating media in the device; *(c)* administering the compound to the tumor cells (*e.g.*, by adding the compound to the media); and then *(d)* quantitatively or qualitatively detecting tumor cells present in the primary chamber and/or the secondary chamber(s), where a lesser number of tumor cells present in the chambers (*e.g.*, as compared to other tumor cells under like conditions but without the administering of the compound of interest) indicates greater anti-metastatic activity and/or anti-tumor activity of the compound of interest. In some embodiments, the tumor cells are collected from a patient afflicted with the tumor. In some embodiment, the method further includes administering the compound to the patient in a treatment-effective amount when the compound is determined to decrease the metastasis of the tumor cells *in vitro*, decrease construct size *in vitro*, decrease the number of tumor cells *in vitro* and/or induce tumor cell death *in vitro.*

In some embodiments, a method of the present invention may comprise screening a compound of interest *in vitro* for anti-metastatic activity and/or anti-tumor activity, the method comprising: *(a)* providing a device as described herein; *(b)* circulating a growth medium from a first chamber comprising a liver organoid to a second chamber comprising a live tumor cell organoid; *(c)* administering a compound of interest to the liver organoid and/or the live tumor cell organoid (*e.g.*, by adding the compound to the growth medium); and *(d)* determining a decrease in the presence of tumor cells (e.g., a change in number or density) in the second chamber, as compared to the number of tumor cells present in the live tumor cell organoid when the test compound is not administered. One or more additional organoids, such as, but not limited to, brain, colon, lung, endothelium, cardiac, epithelial, etc. organoids, optionally containing benign and/or tumor cells may be present in one or more additional chambers of the device, the chambers being in fluid communication with each other.

In some embodiments, a method of the present invention may comprise screening a compound of interest *in vitro* for anti-metastatic activity and/or anti-tumor activity, the method comprising: *(a)* providing a device as described herein (e.g., a device comprising a primary chamber comprising a live tumor cell organoid and at least one secondary chamber comprising a different organoid (e.g., an organoid comprising benign cells)); *(b)* circulating media in the device; *(c)* administering the compound to the tumor cells (*e.g.*, by adding the compound to the media); and *(d)* determining a decrease in the presence of tumor cells (e.g., a change in number or density) in the primary chamber (e.g., associated with the live tumor cell organoid) and/or secondary chamber(s), as compared to the number of tumor cells present in the live tumor cell organoid and/or secondary chamber(s) when the test compound is not administered. Example different organoids in one or more secondary chambers may comprise benign and/or tumor cells and may include, but not limited to, brain, colon, lung, endothelium, cardiac, epithelial, liver, etc. organoids.

A method of the present invention may comprise labeling cells (e.g., tumor cells) with a detectable compound, such as, but not limited to, a fluorescent compound (e.g., dye, protein, etc.).

The foregoing and other aspects of the invention are explained further in the following examples.

### Examples

### Example 1

The first objective of this project was to evaluate a 3D model of a colorectal cancer tumor surrounded by normal host tissue. The engineered concentric ring construct consists of a colorectal tumor core and a colon epithelial outer ring, to model a tumor in a reductionist, yet physiologically relevant environment. This construct was used to measure the growth of the tumor core, any observed invasion of the tumor cells into the epithelial layer, and the efficacy of drugs. The constructs were created with cells suspended in a hyaluronic acid and gelatin-based hydrogel. The tumor cores were made using one of four colorectal cancer cells types, thereby providing models of 4 levels of malignancy. The HCT116 cells were the most malignant, followed by the HT29, SW480, and Caco2 cells. Caco2 cells were also employed in the outer ring, as they best resemble healthy epithelial cells. The tumor core was fluorescently labeled and fluorescent imaging was used to monitor the tumor core over the course of 7-14 days. After this amount of time, the tumor core was shown to become denser, although little invasion was seen. Ring constructs made with a Caco2 outer ring and a SW480 core were also treated with Regorafenib, a chemotherapy agent. An MTS assay confirmed a decrease in the amount of viable cells after treatment and LIVE/DEAD staining showed that Regorafenib selectively targeted the inner ring of the construct, rather than the less tumorigenic Caco2 cells.

The second objective was to develop a system to quantify the number of cells metastasizing from a 3D colorectal cancer tumor construct. A microfluidic perfusion device was designed to catch the circulating metastatic cells on a Transwell membrane sealed within the channel in the device. The device was created using soft lithography of polydimethylsiloxane (PDMS). Tumor constructs consisting of a single tumor cell type suspended in hydrogel was placed in the appropriate chamber, and the device was sealed and clamped. Media was circulated throughout the device and images were taken every 2-3 days for close to 2 weeks. After two weeks, cells were observed on the Transwell membrane indicating that the device is able to support quantification of tumor cells that had entered circulation from the tumor construct. Future studies will be carried out to identify whether tumor cells of different levels of malignancy display different quantitative migration numbers.

In summary, these 2 models - a 3D concentric ring tumor model and the microfluidic circulating tumor cell quantification device - provide new approaches to study growth of tumor cells, the effect of drugs on tumor cell response, and quantification of circulating cells.

### Example 2

3D tumor organoids were microengineered directly from fresh tumor biopsies to provide a patient-specific model system with which treatment optimization can be performed before initiation of therapy. Here, the initial implementation of this platform is demonstrated using a mesothelioma tumor biospecimen removed from a patient. Shown is the ability to biofabricate and maintain viable 3D tumor constructs within a tumor-on-a-chip microfluidic device. Second, it is demonstrated that on-chip chemotherapy screening mimics patient response to the drugs cisplatin and pemetrexed. Finally, it is demonstrated *in vitro* the effectiveness of a candidate compound identified through genetic biomarker assessment.

### Methods

### Thin film microfluidic device fabrication

The microfluidic device fabrication approach is based on methods reported elsewhere. Channels were formed in double-sided adhesive film using a cutting plotter (GraphTec-CE6000-40) and the bottom surface of the patterned layer was adhered to a clean glass microscope slide. On the top surface was adhered a polystyrene slide (Tedpella.Inc) featuring holes produced by drill press to act as inlets and outlets for fluid delivery to the channels. Following device assembly, PTFE tubing was connected to each port and secured using a UV cure polyester resin.

### Patient clinical history.

The patient is a 50 year old male who presented with debilitating cachexia, ECOG 2 functional status and abdominal distention. A diagnostic laparoscopy was performed for tissue diagnosis that established the diagnosis of epithelioid malignant peritoneal mesothelioma. Precision medicine analysis of his tumor revealed two genomic alterations (BAP1 splice site 1729+1G>A and PBRM1 N258fs*6) with absence of available targeted drugs or clinical trials. Computerized tomography (CT) with iv contrast demonstrated voluminous malignant ascites without evidence of disease outside the peritoneal cavity. He was evaluated for cytoreductive surgery(CRS) with heated intraperitoneal chemotherapy (HIPEC). Due to the excessive volume of disease, a complete CRS was unlikely to be achieved at that point. Therefore he was referred for upfront systemic chemotherapy aiming for disease volume downstaging. He was treated with 6 cycles of cisplatin-gemcitabine followed by a single cycle of carboplatin-gemcitabine due to concerns of cisplatin associated ototoxicity. He showed an excellent clinical response to cisplatin based chemotherapy, with almost complete resolution of his malignant ascites both on repeat staging CT and clinical exam that was performed prior to his carboplatin based chemotherapy. Subsequently, he was taken to the operating room and underwent CRS/cisplatin based HIPEC with an unremarkable postoperative course. During surgical exploration and prior to HIPEC, a segment of his omental disease was cut with scissors and was placed in ice and transferred fresh to the lab for tumor organoid development.

### Tumor biospecimen procurement and cell processing

The tumor was delivered within one hour of removal to the lab for cell processing. Once received, tumors were washed in Phosphate Buffer Solution (PBS) with 2% penicillin-streptomycin for three 5 minute cycles and then washed in Dulbecco's Modified Eagle's Medium (DMEM) with 2% penicillin-streptomycin for two 5 minute cycles. Tumors were individually minced and placed into DMEM with 2% penicillin-streptomycin and 10% collagenase/hyaluronidase for 18 hours on a shaker plate in 37C (10X Collagenase/Hyaluronidase in DMEM, STEMCELL Technologies, Seattle, WA). Digested tumors are then filtered through a 100µm cell filter and centrifuged to create a pellet. Plasma and non-cell material was removed and the pellet was re-suspended in 1mL BD PharmLyse with 9mL deionized water for 5 minutes (BD PharmLyse, San Diego, CA). The conical was filled to 50mL with deionized water and centrifuged, lyse buffer with lysed cells was aspirated and the cell pellet was counted for use.

### Extracellular matrix hydrogel preparation and basic organoid formation

The ECM-mimicking HA/gelatin-based hydrogel (HyStem-HP, ESI-BIO, Alameda, CA) was prepared as previously described.^{15,18,27} Briefly, a thiolated HA component (Glycosil) and a thiolated gelatin component (Gelin-S) were dissolved in sterile water containing 0.05% w/v of the photoinitiator 2-Hydroxy-4'-(2-hydroxyethoxy)-2-methylpropiophenone (Sigma, St. Louis, MO) to make 1% w/v solutions. The polyethylene glycol diacrylate (PEGDA) crosslinker (Extralink, ESI-BIO) was dissolved in the photoinitiator solution to make a 2% w/v solution. Glycosil, Gelin-S, and Extralink were then mixed in a 2:2:1 ratio by volume, respectively. For tumor construct formation, the resulting solution is mixed thoroughly by vortexing, used to resuspend cells (20 million cells/mL), after which ultraviolet light exposure (365 nm, 18 W cm⁻²) for 1 s initiates crosslinking and organoid formation.

### Tumor-on-a-chip biofabrication

Tumor constructs were biofabricated in the microfluidic devices using a photopatterning technique similar to our previous work.¹⁵ Here, a photomask featuring 1-3 mm apertures was produced in an aluminum foil with the same cutting plotter technique used for channel definition and adhered to the bottom surface of the microfluidic device with a matching patterned adhesive film. Hydrogel precursor solutions were then mixed with cells derived from the patient mesothelioma biopsy at a density of 20 million cells/mL. The mixture was introduced to each of the device channels via the inlet ports and organoid constructs were defined by ultraviolet light exposure (365 nm, 18 W cm⁻²) for 1 s through the attached photomask. This exposure initiated a rapid thiolene stepwise crosslinking reaction that encapsulated cells in a 3D column. Finally, unexposed precursor/cell mixture was flushed from the device with phosphate buffered saline, leaving a 3D patient-derived mesothelioma construct within each channel. Each channel of the device with the cell construct was connected to separate reservoir with DMEM. The media was flowed through the channel from the reservoir by silastic tubing connected to MP2 Precision micro-peristaltic pump (Elemental Scientific, Inc., Omaha, NE). Flow was initiated and maintained at 10 µL/min.

### Histological assessment

5 *µ*m thick organoid sections were created from paraffin-embedded constructs, and then deparaffinized for staining. IHC was used to visualize biomarkers cytokeratin 5/6 (CK5/6), calretinin, and thrombomodulin. Blocking was performed by incubation under Dako Protein Block for 15 minutes. Primary antibodies CK5/6 (Abcam, ab17133, raised in mouse) and calretinin (Abcam, ab702, raised in rabbit) or CK5/6 and thrombomodulin (Abcam, ab109189, raised in rabbit) were applied to the sections on the slides at a 1:200 dilution in Dako Antibody Diluent and incubated at room temperature for 1 hour. Next, secondary Alexa Fluor 488 or Alexa Fluor 594 antibodies with appropriate species reactivity were applied to all samples at 1:200 in Dako Antibody Diluent and left at room temperature for 1 hour (anti-mouse Alexa Fluor 488 and anti-rabbit Alexa Fluor 594, Life Technologies, Carlsbad, CA, A-11070). Sections were then incubated with Dapi for 5 minutes prior to coverslipping. For Annexin V and Ki67 staining (Abcam, Cambridge, MA, ab14196 and ab16667, respectively) in subsequent biomarker-driven experiments, an identical protocol was employed. Fluorescence images were taken using a Leica DM400B Compound Microscope and overlaid for analysis.

### Cisplatin, carboplatin, andpemetrexed drug study

At the end of first week, LIVE/DEAD staining was performed as mentioned below in two constructs to assure that the cells were alive prior to addition of drug. Two drug combinations were considered to test on this cancer model, Cisplatin+Pemetrexed and Carboplatin+Pemetrexed in two different concentrations of 0.1µm and 10µm mixed in DMEM. The DMEM reservoir was replaced by 2 mL volume of this drug mixture.

LIVE/DEAD staining (LIVE/DEAD Viability/Cytotoxicity Kit for mammalian cells, Life Technologies, Grand Island, NY) was performed on day 7 at the end of the drug exposure period. Circulating media was first flushed from the device channels with clean PBS and then a 1mL mixture of PBS and DMEM (1:1) containing 2 *µ*M calcein-AM and 2 *µ*M ethidium homodimer-1 was introduced. Constructs were incubated for 60 min after which the channels were again flushed with clean PBS. *In situ* imaging was performed using an Olympus FluoView^{™} FV1000 confocal microscope. 5 µm z-stacks were obtained for each construct using filters appropriate for both red and green fluorescence and then overlaid.

Confocal images were processed using Imaris MeasurementPro (Bitplane, Concord, MA). Through the software, images in each color channel were analyzed for cell size, shape, and fluorescence intensity and cell locations were marked. For consistency, a 450 µm × 900 µm area of each image was considered. Cell viability was calculated through quantification of the total number of identified cells in the green channel (LIVE) compared to the total number in the red channel (DEAD).

### Biomarker-driven experimental drug study

The patient from whom the tumor biospecimen was attained was entered into the Precision Medicine Program at the Comprehensive Cancer Center at the Wake Forest Baptist Medical Center, resulting in part of the biopsy being sent for identification of potential actionable mutations in the tumor. Deidentified data indicated a positive mutation in the BAP1 gene, indicating an opportunity to test an enhancer of zeste homolog 2 (EZH2) inhibitor as an experimental, targeted therapy.

Tumor constructs were biofabricated once more, after which they were maintained for 3 days prior to drug screening. Patient organoids were then subjected to the drug 3-deazaneplanocin A (DZNep), a histone methyltransferase EZH2 inhibitor, at concentrations 0.1 µM, 1 µM, and 10 µM in cell culture media for an incubation periods of 96 hours. Organoids biofabricated using HCT116 cells, a colorectal cancer cell line shown to respond to EZH2 inhibition, were tested in parallel as a positive control. Drug effects were quantified by MTS assays and immunohistochemistry. MTS assays (CellTiter 96 One Solution Reagent Promega, Madison, WI) determined relative cell number by quantifying mitochondrial metabolism. Absorbance values were determined on a Molecular Devices SpectrumMax M5 (Molecular Devices) tunable plate reader system at a wavelength of 490 nm.

Immunohistochemisty staining was performed using biomarkers Annexin V and Ki67 on 5µm thick organoid sections. Sections were taken from previously unstained organoids that were embedded in paraffin, sectioned, and then deparaffinized for IHC staining. Primary antibody Annexin V was applied to the slides at a 1:200 concentration in Dako Antibody Diluent and left at room temperature for 60 minutes (Annexin V, Abcam, Cambridge, MA, ab14196). In parallel on separate samples, primary antibody Ki67 was applied at a 1:200 concentration in Dako Antibody Diluent (Ki67, Abcam, ab16667). Secondary Alexa 488 antibodies were applied to all samples at 1:200 in Dako Antibody Diluent and left at room temperature for 1 hour (Alexa 488, Life Technologies, Carlsbad, CA, A-11070). Fluorescence images were taken using a Leica DM400B Compound Microscope, images were taken of sections next to each other in GFP for Annexin V and Ki67 and overlaid for analysis; Annexin V images were colored red (medium grey in black and white images) and Ki67 images were colored green (light grey in black and white images).

### Results

### Precision medicine driven tumor organoid strategy

Our goal has been to develop a platform consisting of patient-specific tumors in which anticancer drugs will be tested for efficacy, which can then inform treatment optimization. The concept of personalized precision medicine works as follows. When a tumor biopsy is taken from a patient, some or all of the biospecimen is genetically sequenced in part or in full in order to identify mutations that may indicate drugable targets. Subsequently, if FDA-approved drugs are available or clinical trials are ongoing that target one of those biomarkers, the therapy for that given patient will be adjusted accordingly (**Fig. 1** **- solid arrows**). Under optimal conditions, this strategy results in therapies that are more effective at standard chemotherapy. However, in practice, often multiple mutations are identified, resulting in a panel of potential drug options, with no clear best treatment, necessitating additional tools to test these drugs, as well as potential experimental drugs. Our approach is to integrate patient-derived tumor organoids, formed using the same biospecimen materials sent for genetic profiling. In this methodology (**Fig.** 1 - **dashed arrows**), portions of the biospecimens are employed to biofabricate numerous micro-scale 3D tumor organoids using a cell supportive extracellular matrix hydrogel biomaterial. These organoids are then employed in drug screens designed to test the agents identified in genetic screens, thereby determining which agent is most effective for that patient.

### Patient-specific tumor-on-a-chip biofabrication

**Fig. 2** **panels A-C** describes the process for fabricating thin film microfluidic devices, integration of tumor organoids *in situ*, and general device operation. Devices capable of housing 6 independent tumor organoids are comprised of an adhesive film in which fluid channels and organoid chambers are cut, which is sandwiched between a polystyrene slide with inlet and outlet ports and a glass slide with an attached photomask **(****Fig. 2****, panel A**). Tumor organoids were patterned using hydrogel precursor solution containing the patient tumor-derived cells, which was introduced into each of the 6 channels, and photopolymerized as organoids through the photomask apertures **(****Fig. 2****, panel B**). After organoid biofabrication, the devices were connected to a micro-peristaltic pump and media reservoirs via tubing, after which flow was initiated for organoid culture **(****Fig. 2****, panel C**).

Initially, each tumor construct was left under circulating DMEM for seven days or 14 days. To verify the ability of the platform to maintain viable patient-derived tumor models, we performed LIVE/DEAD analysis on organoids at day 7 and day 14 **(****Fig. 2****, panel D**). The results indicated high cell viability, greater than 90% on day 7 and greater than 85% on day 14, thereby demonstrating that our device architecture and the supporting hydrogel matrix can support patient-derived cells for extended experimentation.

### Tumor-on-a-chip supports viable patient-derived models and chemotherapeutic drug studies correlate with patient drug response

On day 7, a subset of tumor constructs were exposed to one of two different doses each of chemotherapeutic mixtures carboplatin/premetrexed and cisplatin/premetrexed, or no drug. Following a seven day treatment, LIVE/DEAD analysis was performed on all organoids (**Fig. 3****, panels a(i)-f(i) and a(ii)-f(ii))**. Under control (no drug) conditions, tumor constructs were observed to maintain high viability, showing a statistically insignificant decrease to 87% after a total of 14 days in the device. However, organoids exposed to drug mixtures showed a considerable reduction in live cell ratio. For samples treated with carboplatin/premetrexed, we found viabilities reduced to 52.1% for a circulating concentration of 0.1 µM and to 39.8% for 10 µM. While the high dose was two orders of magnitude greater concentration than the low dose, the observed change in viability had only low significance (p<0.1), indicating a potentially moderate effect for the drug. In contrast, organoids treated with cisplatin/premetrexed yielded decreases in viability to 39.0% for 0.1 µM concentration and to 11.8% for 10 µM. These represented significant declines relative both to each other (p<0.05) and to control measurements (p<0.05 and p<0.001, respectively). Consequently, cisplatin/premetrexed was significantly more effective than carboplatin/premetrexed in our device. These results are summarized in **Fig. 3****, panel g**.

These data have two central outcomes. First, they demonstrate that cells derived from a patient tumor biopsy can be sustained in a bioengineered organoid system long-term for extensive study. To our knowledge, this is the first report of this capability. Second, they show that *in vitro* drug screening of such organoids is feasible, yielding drug-dependent reductions in cellular viability. This represents a potential asset to therapy design, as drug efficacy could in principle be established in a patient-specific manner prior to administration of treatment. For example, in these measurements, our results would suggest cisplatin/premetrexed as a superior treatment option to carboplatin/premetrexed. The nature of personalized medicine prevents a systematic comparison of the effectiveness of the two agents in the patient. However, crucially, the patient was treated with cisplatin/premetrexed with excellent response. This suggests that patient-specific drug effectiveness may be recapitulated accurately in our *in vitro* system.

### Experimental drug screening driven by genetic biomarker identification

Precision Medicine testing identified two known mutations in the tumor biospecimen: *BAP1* splice site 1729+1G>A and *PBRM1* N258fs*6. *BAP1* (BRCA1 associated protein-1) is a deubiquinating enzyme²⁸ while *PBRM1* is a tumor suppressor gene associated with several cancers.²⁹ Neither mutation was associated at the time of testing with a FDA-approved therapy in either mesothelioma or any other tumor type.

However upon further investigation, we identified that mutations of *BAP1* have been targeted with success in animal models and *in vitro* studies by inhibition of EZH2,³⁰ an enzyme that participates in DNA methylation and transcriptional repression **(****Fig. 4****, panel A**). Therefore, we performed a drug screening experiment using the EZH2 inhibitor DZNep. As a control population, organoids were generated using the widely studied colorectal cancer cell line HCT116. Importantly, HCT116 cells have been shown to undergo cell cycle arrest, but not necessarily significant levels of cell death, upon treatment with DZNep,³¹ and as such we deemed them an appropriate control for this study.

Our results show that after drug treatment, there was no significant decrease in HCT116 proliferation but a significant decrease in mesothelioma proliferation **(****Fig. 4****, panel B**). Although the controls for HCT116 and mesothelioma were similar, the DZNep treated mesothelioma showed a decrease in proliferation in comparison to the control and with increase in drug concentration. As DZNep concentration was increased, cell proliferation decreased. Additionally, comparison between HCT116 and mesothelioma, show significantly lower mesothelioma cell proliferation for all treatment concentrations. Annexin V and Ki67 immunohistochemistry was carried out for both cell types over all conditions. Annexin V appears to increase in mesothelioma with increase in DZNep concentration representing an increase in apoptotic cells **(****Fig. 4****, panel C**). However, minimal change in proliferation is seen across all HCT116 conditions indicating DZNep did not greatly affect proliferation **(****Fig. 4****, panel D**). Hematoxylin and eosin staining was done on each of the organoids and representative images of mesothelioma show ghost cells and those without nuclei when treated with DZNep **(****Fig. 4****, panel E**). This response is not seen in mesothelioma or HCT116 controls and is not present in any HCT116 treatments (**Fig. 4****, panel F**). The mesothelioma only response further indicates that DZNep has measurably reduced proliferation of the patient sample and that HCT116 has acted as an appropriate positive control with no response.

### Discussion

Precision oncology, whereby tumor DNA is sequenced to identify actionable gene mutations, is poised to become a standard clinical practice for therapeutic decision making of cancer treatment. However, currently, any reported correlation between a genetic alteration and a potential drug cannot be verified prior to initiation of treatment, while only 11% of precision medicine tested patients will follow a precision medicine guided treatment. Moreover, there is a great variability in the biologic behavior of cancer based on histologic type, grade and volume of disease.

Within research, patient derived xenografts (PDX) have been used to study patient tumor progression and drug treatment response. These models are lacking in that they require immunodeficient mice to place the biopsies or tumor samples in which causes them to become infiltrated with cells from the mouse. The cells also adapt to their new environment and genetic drift has been shown from the initial samples again making them less ideal. One additional limiting factor in widespread adoption of PDX technology is the fact that only the most malignant of tumor biospecimens successfully "take" when introduced into immunodeficient mice. This poor take rate, generally agreed to be in the 25 - 33% percent success rate range, limits the applicability of PDX technology and its predictive applications to the majority of cancer patients. In beginning the work described herein, and in parallel ongoing studies, a major critique of our approach was that could we improve upon the take rate percentage of PDX. Remarkably, this has been surprisingly straightforward in our platform. As is shown in **Fig. 2**, we see high cell viability at day 7 and day 14 of culture. Moreover, as we are performing similar studies with other mesothelioma biospecimens and other GI tumor biospecimens, we have observed a take rate above 90%. It is important to mention, that the 10% non take rate, possible reflects accelular specimen obtained from low grade mucinous appendiceal patients. The above stems from the observation that as of today all high grade specimens grew organoids. This is significant, and indicates to us that the use of 3D, ECM microenvironment-supported organoid technology may be able to create deployable models for many more patients than PDX technology is capable of. While incredibly promising, this further evidence of take rate efficiency will require significant additional studies to confirm.

In this collection of experiments, beyond take rate and viability assessment, we demonstrate feasibility of two additional proof-of-concept scenarios: 1) patient versus tumor organoid correlation; and 2) biomarker-driven testing of an experimental drug. First, we employed the mesothelioma organoid platform to screen two of the common chemotherapy regimens used clinically for this type of cancer. Specifically, we tested organoid response to the combination of cisplatin and pemetrexed versus carboplatin and pemetrexed (**Fig. 3**). Exposure to the drug for 7 days through infusion into the circulating media via the microfluidic system resulted in a distinct result indicating that the cisplatin-pemetrexed treatment was more effective. The ratio of dead to viable cells was higher in the treatment of 10 µM cisplatin and pemetrexed compared to the comparative concentrations of carboplatin and pemetrexed. On its own, this result validates our platform in the sense that we can successfully perform drug screening studies with it. However, more importantly, the patient from which these organoids were derived, also responded dramatically to cisplatin based chemotherapy as demonstrated by almost complete resolution of his voluminous ascites on CT imaging performed at the conclusion of his cisplatin based chemotherapy. As a matter of fact the patient would not have been an operative candidate unless his disease had responded to cisplatin based treatment. This is significant, and these kinds of correlative results between patients and our organoid technology is a dataset that will be important to continue to grow in order to validate this methodology for clinical adoption.

Second, we were made aware of the genetic testing results using a portion of the patient's tumor biospecimen. This report indicated that two mutations were identified specific to this tumor *- BAP1* splice site 1729+1G>A and *PBRM1* N258fs*6. As described above, *BAP1* is a deubiquinating enzyme²⁸ while *PBRM1* is a tumor suppressor gene associated with several cancers.²⁹ At the time of these studies, neither mutation was associated with a FDA-approved therapy in either mesothelioma or any other tumor type, nor are any pertinent clinical trials under way. However, we did learn that mutations of *BAP1* have been targeted in animal models and *in vitro* studies by EZH2 inhibition.³⁰ Therefore, we performed a drug screening experiment using the EZH2 inhibitor DZNep as a test scenario to query whether these biomarkers indicated in the genetic analysis could indeed be used as actionable targets (**Fig. 4**). Indeed, upon treatment with this compound, we observed a decrease in mitochondrial metabolism, a metric generally proportional to cell number. Additionally, we observed an increasing trend in annexin V expression, indicating apoptosis, versus a decreasing trend in Ki67 expression, which identifies proliferating cells. Moreover, H&E staining show that cell morphology and intact cell nuclei suffer in a dose dependent manner with the compound. Organoid created in an identical fashion from the widely used HCT116 colorectal cancer cell line did not respond in the same fashion. Rather, DZNep appeared to have little effect between regardless of the concentration.

By implementing these 3D tumor models in a parallelized microfluidic platform with a circulatory system, we not only have the capability to assess multiple drug types and doses onboard a single device, but we also gain the opportunity to visualize and track the kinetics of tumor progression, migration, and intravasation into circulation, which occurs prior to metastasis to a distant site.

### Example 3

Multiple sets of patient tumor-derived organoids have been generated with over a 90% take rate *in vitro* (versus below 10% in 2D cell cultures), which is favorably compared to a reported 25-33% take rate in patient-derived xenograft (PDX) models where only the most aggressive biospecimens prosper. This poor take rate limits the application of PDX technology to predictive diagnostics for most cancer patients. Our tumor organoid systems have subsequently been employed in a drug screens to demonstrate that like patients, their tumor organoids maintain selective response to different drugs. To date sets of patient-specific organoids have been created from a diverse group of primaries and their metastatic sites including colorectal cancer, high and low grade appendiceal metastatic sites to omentum ovary and liver, peritoneal mesothelioma, and extremity sarcoma. Described herein are several examples, demonstrating 1) correlation between tumor organoid drug response and the drug response of the patient from which the organoids were derived, 2) the capability to test the efficacy of therapies designed based on identification of druggable mutations in the patient's tumor, and 3) establishing viable organoids from difficult to culture tumor populations.

We have demonstrated the capability to create viable tumor organoid models from rare and difficult to isolate and culture cancer types. For example, we have created viable patient-specific tumor organoids from low grade appendiceal (LGA) tumors (**Fig. 5****, panel A**) and well-differentiated papillary mesothelioma (**Fig. 5****, panel B**) - traditionally referred to as "benign" mesothelioma - both of which have slow proliferation patterns and unpredictable transformation potential. Establishing cell cultures to date from primaries with low proliferation index has essentially been impossible. Importantly, our LGA organoids do not respond to chemotherapeutic agents (**Fig. 5****, panel C**) compared to high grade organoids and 2D cultures that do respond to treatment. These data may be the first available hard evidence that LGAs are indeed chemoresistant tumors and therefore treatment of LGA patients with chemotherapy is not beneficial, sparing them unnecessary toxicity. Furthermore, we recently processed an extremity sarcoma and created sets of patient specific sarcoma organoids that also express high viability in our platform (**Fig. 5****, panel D**). These sarcoma organoids are currently being employed in drug screening studies.

### Example 4

The restriction of a normal cell type to malignant tumor cells is maintained by alterations to cell-to-cell interactions and by physical barriers (Lodish H, Berk A, Zipursky SL, et al., 2000). Anticancer drugs travel to the tumor via the circulatory system and must penetrate through the extravascular tissue to successfully target the cancer cells residing within organs (Tannock et al., 2002). The ringed-organoid model attempts to replicate the *in vivo* process of anticancer drug delivery by creating simple 3D micro-organs, or organoids, that contain an invasive cancer type surrounded by a different cell type, and then using specific anticancer drugs to target the inner, invasive cell line. In addition, experiments described herein were performed to determine whether the anticancer drug successfully targets the inner, more invasive cell type (effectiveness determined from previous 3D drug screen) as opposed to the outer cell type when both are present in a 3D microenvironment similar to that found *in vivo.*

In order to investigate the influence of the extracellular microenvironment on the efficacy of anticancer drugs, various concentrations of anticancer drugs were administered (Regorafenib, Sorafenib, Trametinib, 5- Fluorouracil, and Dabrafenib) to four colorectal cancer cell lines (HCT116, HT29, SW480, and CACO2) while they were embedded within hyaluronan-based hydrogel or seeded directly into the wells of a well-plate. The colorectal cancer cells that were embedded within the hyaluronan-based hydrogel simulated cancer cells in an *in vitro* three-dimensional microenvironment and the cells seeded directly into the wells represented cancer cells grown in a traditional two-dimensional microenvironment.

### 2D Tumor Construct Drug Screen

2D drug screens with the colorectal cancer cell lines present in a monolayer culture were conducted to determine anticancer drug efficacy in conventional drug screen practices that lack the cell-to-ECM interactions typically found in malignant tumors in vivo.

Cancer cells seeded directly into wells were given 4-5 hours to reattach to the bottom of the wells. After that time, the anticancer drug solutions were administered in place of the media (**Fig. 6****, panel A**) and an MTS assay was carried out 2 days later to determine cell viability. MTS absorbance results for the anticancer drugs are displayed relative to that of the control, no drug, MTS value (**Fig. 6****, panel B**). At 100 µM concentrations of Regorafenib and Sorafenib, and 100 mM concentrations of 5-FU, cellular viability remained below 20% that of the control for all four of the colorectal cancer cell lines utilized. However, screening with Dabrafenib and Trametinib produced diverging results from the other three anticancer drugs at higher concentrations. 100 µM concentrations of Dabrafebib resulted in relative cellular viability values ranging from approximately 36% for HCT116 cells to approximately 93% for SW480 cells, and 100 nM concentrations of Trametinib resulted in relative cellular viability values ranging from approximately 39% for CACO2 cells to approximately 58% for SW480 cells. When treated with 1mM concentrations of 5-FU, relative cellular viability was approximately 39% for SW480 cells and remained between 10%-22% for the HCT116, HT29, and CACO2 cells. These values were considerably lower than the relative cellular viability values for the lowest concentrations of the other anticancer drugs.

### 3D Tumor Construct Drug Screen

3D drug screens were conducted on the colorectal cancer cell lines while embedded within hyaluronan-based hydrogel constructs to observe how cell-to-ECM interactions in the presence of hyaluronic acid can affect the efficacy of the anticancer drugs.

Polydimethylsiloxane, or PDMS, was used to coat the bottom of the wells. Cells were embedded within the hyaluronan-based hydrogel and were given 5 days to acclimate to their three-dimensional environment and on day 5 were administered different anticancer drug solutions (**Fig. 7****, panel A**). An MTS assay was conducted on day 7 and results were displayed relative to that of the control values (**Fig. 7****, panel B**). At 100 µM concentrations of Regorafenib and Sorafenib, cellular viability remained below 25% that of the control for all four of the colorectal cancer cell lines utilized. The only exception to this was when 100 µM Regorafenib was administered to CACO2 cells and the percent relative cell viability rose to approximately 48%. Screening with 100 mM 5-FU resulted in relative cell viability values between 48% when used on SW480 cells and 81% when it was administered to HCT116 cells. However, the 10 mM concentrations of 5-FU only resulted in cellular viability values for all four cell lines in a range between 9% for SW480 cells and 50% for HT29 cells. At a 100 µM concentration, Dabrafenib had greater efficacy in targeting the HCT116 and SW480 cell lines, which had relative cellular viability values of 38% and 43%, as opposed to the HT29 and CACO2 cell lines, which had higher cell viability values of 93% and 75%. 100 µM concentrations of Trametinib produced relative cellular viability results that fell within a narrow range for the four colorectal cell lines with values of 54% for SW480 cells and 74% for HT29 cells.

### 2D and 3D Drug Screen Comparison

Significant difference statistics were carried out on the 2D and 3D drug screens to establish whether the colorectal cancer cells reacted differently to the anticancer drugs when in either environment. Significant differences in cellular responses to the drugs in these drug screens would suggest that the cell-to-ECM interactions between the cells and hyaluronic acid are to blame. HCT116 and CACO2 responses to Regorafenib, Sorafenib, and 5-Fluorouracil are due to morphological differences and similarities between these cell types. These cells and drugs will be used in later sections of this study.

Although the same cell lines were provided identical concentrations of the anticancer drug solutions, it is apparent that the cells responded to the drugs treatments differently when placed within a two-dimensional versus three-dimensional in vitro microenvironment. Significant differences in cell viability were observed between the results of the two- and three-dimensional drug screens using Regorafenib, Sorafenib, and 5-Fluorouracil on HCT116 and CACO2 (**Fig. 8**). For the HCT116 cells, two results were significantly different between the 2D and 3D screens with Regorafenib (both <0.05), one was significantly different for Sorafenib (<0.10), and two were significantly different for 5-FU (both <0.01). For the CACO2 cells, two results were significantly different between the 2D and 3D screens with Regorafenib (both <0.01), three were significantly different for Sorafenib (two <0.01 and one <0.05), and three were significantly different for 5-FU (two <0.01 and one <0.10).

For the HCT116 cells, apart from the 10 µM solution of Sorafenib and the 10 mM solution of 5-FU, 3D drug screens resulted in higher relative cellular viability values than the 2D drug screen. For the CACO2 cells, apart from the 10 µM solutions of Regorafenib and Sorafenib and the 10 mM solution of 5-FU, the 3D drug screen also resulted in higher relative cellular viability values than the 2D drug screen. Comparing the 2D and 3D drug screen (**Fig. 8**) results conveys that Regorafenib had a more significant impact on altering cell viability in HCT116 cells than in CACO2 cells, that Sorafenib had a more significant impact on altering the cell viability of CACO2 cells than HCT116 cells (although HCT116 was already greatly affected in both screens), and that 5-FU significant altered the cell viability of both the HCT116 cells and the CACO2 cells. During the 3D drug screen, at 100 µM concentrations of Regorafenib and Sorafenib, HCT116 cells had lower percent relative cell viability values than CACO2 cells at 21% as opposed to 48%, and 15% as opposed to 25%. Since the 100 mM concentration of 5-FU produced viability values far higher than those during the 10 mM 5-FU drug screen, when looking at the 10 mM 5-FU concentration results for both the HCT116 cells and the CACO2 cells in 3D microenvironments, it is clear that their relative cell viability was lowered to fairly similar values at 18% and 22%.

### Ringed Organoid Drug Screening

The ringed-organoid studies tested whether cell-to-ECM interactions or cell-to-cell interactions between different cell types within a 3D hyaluronan-based hydrogel microenvironment affected the efficacy and specificity of the anticancer drugs in targeting certain cancer cells, as previously observed in the 3D drug screen.

Polydimethylsiloxane, or PDMS, was again used to coat the bottom of the wells. HCT116 cells were embedded within hyaluronan hydrogel and constructs were created on the PDMS surface. CACO2 cells that were also embedded within hyaluronan gel were used to create the outer ring around the HCT116 inner-ring construct (**Fig. 9****, panel A**). Since HCT116 cells displayed lower relative cell viability values when administered 100 µM concentrations of Regorafenib in comparison to CACO2 cells in a 3D microenvironment (21% versus 48%), and because 5-FU showed similar effects on decreasing relative cellular viability in a 3D microenvironment, these drugs were administered in equivalent concentrations (1 µM, 10 µM, and 100 µM) to ringed-constructs to observe and quantify the efficacy of these drugs on the two cell lines for comparison.

A live-dead stain was carried out on the ringed-constructs on day 7 and imaged on an Axiovert microscope (**Fig. 9****, panel B**). The imaging software, MATLAB, was used to generate a % dead: % live ratio (or % red (medium grey in black and white images): % green (light grey in black and white images)) for the ringed-constructs. The values were then graphed showing the ratios for each of the concentrations of Regorafenib and 5-FU and against the control, untreated ratio (**Fig. 9****, panel C**). At 100 µM, Regorafenib and 5-FU both have % dead: % live ratios of 0.98. This means that approximately half of the HCT116 and CACO2 cells are alive and half have been targeted by the anticancer drugs. When analyzing the relative cellular viability when the 1 µM and 10 µM concentrations of Regorafenib and 5-FU were administered, the % dead: % live ratios are much greater for the 5-FU solutions than for the Regorafenib solutions. At 1 µM, the % dead: % live ratio is 0.95 for 5-FU as opposed to 0.48 for Regorafenib, and at 10 µM, the % dead: % live ratio is 0.78 for 5-FU as opposed to 0.08 for Regorafenib. The % dead: % live ratios for the three concentrations of Regorafenib and 5-FU were substantially higher than the control % dead: % live ratio value of only 0.03.

When looking at the live-dead images in panel B, it appears as though the inner HCT116 cells were affected by Regorafenib to a greater extent than the CACO2 cells, since the majority of the red (medium grey in black and white images) cells appear within the inner ring (right-side of the images) and not the outer ring (left-side of the images). In contrast, 5-FU appears to be targeting both cell types to similar degrees as dead cells that have been stained with the red (medium grey in black and white images) Ethidium Homodimer-1 dye appear in both the inner (HCT116) and outer (CACO2) rings. Moreover, increasing the anticancer drug concentrations by a factor of 10 results in observable increases in targeted cell death by the drugs.

### Microfluidic Device for Quantifying Metastasis

The PDMS-based microfluidic device (**Fig. 10**) was developed to determine how rates of cancer cell metastasis varied when cancer cells were embedded within 3D hyalruonan-based constructs and were administered anticancer drug solutions flowing in a simplified circulatory system. In addition, the microfluidic device sought to determine how the anticancer drugs affected cancer cell viability within the "primary" tumor construct and in "secondary" cancer cells captured on the transwell membrane.

Two microfluidic devices were set up with the intent of running 7 days to observe the device's feasibility in capturing cells on a transwell membrane that have entered into media circulation. 3D constructs with embedded HCT116 cells were implanted within the "construct chambers" of both of the devices. Untreated media was added to the fluid reservoir of one of the devices (control) and 10 µM solution of Sorafenib was added to the fluid reservoir of the other device. Images of the control device's construct and transwell membrane were taken on Days 1 and 7 with a Zeiss Axiovert 200M 487-1 microscope. An increase in captured cells on the transwell membrane of the control device was observed between Days 1 and 7 as indicated by the DiO, green membrane dye. The transwell membrane was imaged on a confocal microscope on Day 7, following device disassembly, and also determined that the device was successful in capturing cells that migrated out of the 3D construct and entered circulation. The flow rate of the device was recorded as 0.20 mL/min.

The device circulating the drug-media solution of 10 µM Sorafenib displayed signs of internal fungal infection on Day 3 and was thus disassembled and discarded following this observation. As a result, any data that could have been collected from this device was lost. A minor leakage from the device's outlet was determined to be the culprit of the contamination.

### Discussion

These studies established that the 3D extracellular microenvironment plays a significant role in determining the efficacy of anticancer drugs. The four colorectal cancer cell lines showed significant differences in cellular viability following embedding and encapsulation in hyluronan-based hydrogel and the administration of five anticancer drugs. The ringed organoid allowed the qualitative comparison of drugs targeting different cancer cells when embedded in a 3D hydrogel environment and suggests that the ringed organoid may provide a way of measuring cell migration. The microfluidic device developed displays the ability to capture metastasizing cancer cells, allows continued microscopic observation of the 3D construct and captured cells, and suggests use as a model and platform for personalized chemotherapy drug screening.

### Materials and Methods:

### Cancer Cell Line Origin

The HCT-116, HT-29, SW-480, and CACO-2 colorectal cancer cell lines used originated from the Cell and Viral Vector Core Laboratory of the Wake Forest University School of Medicine. Cells were cultured in 15 cm cell culture plates and standard DMEM-10 (Dulbecco's Modified Eagle Medium) containing DMEM High Glucose solution (HyClone, Utah, USA), 10% fetal bovine serum, 1% penicillin/streptomycin, and 1% L-glutamine was used as the cell growth medium.

### 2D Drug Screening

Sterilization and Cell Passaging: Media was aspirated from the cell culture plates and 10 mL of PBS (25 °C) was pipetted into each of the plates. The cell culture plates were maneuvered so that the PBS covered all of the cells within the plates and the PBS was then aspirated out of the plates. 5 mL of 0.05% Trypsin (37 °C) was pipetted into each of the cell culture plates. The cell culture plates were then placed in a 37 °C incubator for ~8 minutes. After this time, 5 mL of DMEM-10 media was added to each of the plates. 10 mL of the cell and media components were then placed into four clean 15 mL centrifuge tubes.

Cell Counting and Loading onto Hemocytometer: Cells were resuspended within the 15 mL centrifuge tubes with a 1000 µL single-channel pipette and 100 µL was transferred from each cell line tube into a new 15 mL centrifuge tube. 400 µL of DMEM-10 media and 500 µL of Gibco Trypan Blue Stain (0.4%) (Thermo-Fischer Scientific, Massachusetts, USA) dye was then pipetted into each of them. The second centrifuge tubes served as a 1:10 dilution to hasten cell counting and the addition of Trypan Blue Stain was to exclude dead cells from being included in the cell count. 10 µL of the 1:10 dilution sample was pipetted with a 2-20 µL single channel pipette and loaded into the counting chamber of a previously sanitized hemocytometer. Live cells were counted and calculations were carried out to determine the total number of cells present of each cancer cell line.

Cell Seeding: The original 15 mL centrifuge tubes containing the 10 mL of DMEM-10, cells, and trypsin were placed into a centrifuge. The centrifugation setting was 1500 RPM and at maximal acceleration for 5 minutes. After that time, the media and trypsin mixture was aspirated from the tubes, leaving the cell pellets still intact. 5 mL of DMEM-10 was then pipetted into each of the tubes, resuspending the cells in the process. Calculated volumes of the 5mL cell-media solution were determined to ensure that 25,000 cells of each of the four colorectal cancer cell lines were seeded directly into each of the wells of a polystyrene clear-bottom 96-well plate. 100 µL of DMEM-10 media was then pipetted directly into the wells. The 96-well plates were incubated at 37°C and 5% CO2 for 5 hours to allow the cells to reattach themselves to the bottom of the wells until media was aspirated and anticancer drug solutions were added.

Addition of Anticancer Drugs for Screening: The anticolorectal cancer drugs used in this screening included 5-FU, Regorafenib, Sorafenib, Trametinib, and Dabrafenib (Sigma-Aldrich, Missouri, USA)). High concentration drug stock solutions were created with DMSO. Lower concentration drug solutions were created by mixing the anticancer drug stocks with DMEM-10 media. 1 mM, 10 mM, and 100 mM drug solutions were generated for 5-FU; 1 µM, 10 µM, and 100 µM drug solutions were generated for Regorafenib, Sorafenib, and Dabrafenib; and 1 nM, 10 nM, and 100 nM drug solutions were generated for Trametinib. Media was carefully aspirated from the wells and 200 µL of the various drug-media solutions were placed into the wells containing cells. Three duplicates were generated for each anticancer drug concentration with each of the different cell types to calculate averages and run statistics on the data generated. Well plates were then incubated at 37°C and 5% CO2 for 24 hours.

Cell viability assay: Cell viability was determined 48 hours after exposure to the anticancer drug solutions. The viability of the cells was examined by the CellTiter 96^{®} AQueous One Solution Cell Proliferation (MTS) assay (Promega, Wisconsin, USA). The anticancer drug-media solutions were aspirated from the wells and 200 µL of the MTS reagent was carefully pipetted into the wells. The incubation time for the cells with the MTS reagent was approximately 45 minutes. The cell viability assay was performed on another 96-well plate and the absorbance was quantified by the EnVision Multilabel Plate Reader (PerkinElmer, California, USA).

### 3D Cancer Organoids for Drug Screening

Well Coating with PDMS and Cell Isolation: Polydimethylsiloxane, or PDMS, (DOW Corning, North Carolina, USA) was used at a 1:10 curing agent ratio to coat the bottom of the 96-well plates and the plates were then placed in a 80 °C oven for at least one hour. Cells were passaged in the same methods previously mentioned (A) with the desire to place 10 µL 3D hyaluronan-based hydrogel constructs containing 100,000 cells each (10,000,000/ µL) into the wells of a 96-wells plate for all four colorectal cancer cell types. Depending on the cell count, a desired number of cells were isolated in new 15 mL centrifuge tubes as cell-media solution and underwent centrifugation.

Hydrogel Components of the Single-Cell Type Constructs: The hyaluronic acid-based hydrogel (ESI BIO, California, USA) was produced using a mixture of three components: Heprasil, Gelin-S, and Extralink PEGDA 2-ARM Acrylate crosslinker. These components were mixed in a 2:2:1 ratio by volume. 0.1% Irgacure photoinitiator (Sigma-Aldrich, Missouri, USA) was added to each of the three components prior to their mixture and the three components were placed in a 37 °C incubator for a ~30 minutes to solubilize.

Forming Organoid Constructs: Once solubilized, the media was aspirated from the 15 mL centrifuge tube (post-centrifugation) and the cells were isolated as a pellet. The hydrogel components were added directly into the centrifuge tube containing the cells and the cells were carefully resuspended in the mixture. 10 µL of the gel-cell solution was placed in the center of the wells on the PDMS coating. This was continued for all wells containing cells and was repeated for each cell type. After this point, the gel can be exposed to UV light from a BlueWave 200 spot light (Dymax, Connecticut, USA) to solidify the hydrogel, or one can wait ~30 minutes for the gel to form on its own. DMEM-10 media was then placed in the wells and the constructs were incubated for 5 days.

Anticancer Drug Screening and Cell Viability: The same five anticancer drugs were added on Day 5 and the cell viability assay occurred on Day 7.

### Ringed Organoids for Comparative Anticancer Drug Effects

Hydrogel Components of the Ringed Organoid Constructs: The hyaluronic acid-based hydrogel used in making the ringed organoids was a mixture of four components: Heprasil, Gelin-S, and PEGDA 2-ARM Acrylate crosslinker (Extralink), and Alkyne-PEG-Alkyne 2-ARM crosslinker (ESI BIO, California, USA). These components were mixed in a 4:4:1:1 ratio by volume. 0.1% Irgacure photoinitiator was added to each of the four components prior to their mixture and the three components were placed in a 37 °C incubator for a ~30 minutes to solubilize. HCT116 and CACO2 cells were isolated in the passaging method previously mentioned and PDMS was used to coat the bottom of the 96-well plates.

Forming Ringed-Organoid Constructs: The four components of the hydrogel were added to the 15 mL centrifuge tube containing the HCT116 cell pellet and the cells were resuspended within the mixture. 10 µL of the HCT116 containing hydrogel were placed in the center of the wells and left untouched with aluminum foil covering the 96-well plates for ~35 minutes. During this time, the PEGDA 2-ARM Acrylate will crosslink the gel, while the UV-sensitive 2-ARM Alkyne-PEG-Alkyne will remain uncrosslinked.

While waiting for the PEGDA to crosslink, add the four hydrogel components to the 15 mL centrifuge tube containing the isolated CACO2 cells and resuspend the cells within the gel. Remove the aluminum foil wrapping on the 96-well plate and inspect the construct to see if they have solidified yet. If so, take 25 µL of the CACO2-embedded-hydrogel and carefully place in the area around the HCT116 construct. Use the pipette tip to physically move the CACO2 gel around the construct on all sides. Use UV light from BlueWave 200 spot light to crosslink both gels together to form a fused, ringed-construct. Add DMEM-10 media to fill up the wells.

Anticancer Drug Screening: On day 5, 1 µM, 10 µM, and 100 µM drug solutions of 5-FU were added to ringed constructs and 1 µM, 10 µM, and 100 µM drug solutions of Regorafenib were added to separate ringed constructs. Three duplicates were generated for each of the drug concentrations and a no-drug control was maintained as well.

Live-Dead Staining and Microscopy: On day 7, the ringed-constructs underwent a Live-Dead stain and the construct were imaged on an Axiovert 200M 487-1 microscope (Carl Zeiss AG, Oberkochen, Germany). Ringed constructs were exposed to media containing Calcein AM and Ethidium Homodimer-1 stains (Thermo-Fischer Scientific, Massachusetts, USA) at equivalent concentrations of 1.0 µl/mL. Constructs were washed with PBS twice before and after exposure to the Live-Dead stain solutions. Percent red to percent green ratios were calculated using the software, MATLAB (MathWorks, Massachusetts, USA), to determine the efficacy of the drugs on the HCT116-CACO2 ringed constructs when in the presence Regorafenib and 5-FU. The % red: % green ratio is represents a % dead: % live cell ratio.

### Microfluidic Device

Assembly: Graphtec Studio software was utilized to construct the molds for the novel microfluidic devices. The device molds were etched into double sided tape with aluminum foil attached to one side using a Graphtec ce6000-60 Plotter (Graphtec, Tokyo, Japan) and the molds were then physically attached to cell culture plates. 20 mL of PDMS (1:10 curing agent ratio) was placed into the plates and the PDMS was then placed into a 80°C oven overnight to solidify. The PDMS molds were cut from the plate once the next day.

8.0 µm Isopore Transwell Membrane Filters (Merck Millipore, Massachusetts, USA) were cut to specifically fit the size of the device compartment containing the transwell membrane for cell capture. The transwell membrane was trapped between two PDMS layers by plasma bonding the PDMS layers using a Plasma Cleaner PDC-32G (Harrick Plasma Inc., New York, USA). The device PDMS layers were sterilized prior to their use with 70% ethanol solution in a laboratory hood.

Running: HCT116 cells were passaged and cells were treated for ~20 minutes with DiO membrane dye (Thermo-Fischer Scientific, Massachusetts, USA) at 5 µL/ mL of DMEM-10 media. 10 µL hyaluronan-based hydrogel constructs containing 100,000 HCT116 cells (10,000,000/ µL) were placed into the organoid chamber. PDMS layers were pressed together and plastic clamps were used on either side of the device to hold PDMS layers in place using four screws. 3 mL of untreated media was added to the fluid reservoir. Imaging on the construct and transwell membrane occurred using an Axiovert 200M 487-1 microscope and on Day 7, Confocal microscopy (Leica Camera, Wetzlar, Germany) imaging was carried out on the transwell membrane.

### Example 5 - 5 organoid integrated drug screening

Aim of Experiment: To assess dependence of multi organoid toxicity on liver organoid metabolic function. As an example, the metabolic activity of the liver can heavily influence the outcome and efficacy of some drugs. For instance, 5FU, a common 1^{st} line cancer drug is cytotoxic to many cells in the body, not only tumors. As such, it is usually given in the form of a prodrug, capecitabine, which is essentially inert until it passes through the liver and is metabolized into its active form.

Design and Results of Experiments: Five tissue platforms were initiated containing liver, cardiac, lung, testis, and brain organoids and maintained for 7 days prior to the start of the drug study. At this point, the liver modules were removed from half of the platforms. Capecitabine (20 uM) was administered to all platforms after which viability was assessed through live/dead staining or cardiac beating behavior after 7 days of exposure (day 14 of total study).

The results demonstrated that with liver present, capecitabine is metabolized into the toxic drug 5-FU, causing heart and lung toxicity (**Fig. 11**). Without liver, this metabolism does not occur, and cardiac and lung organoid viability does not decrease. Surprisingly, brain organoids suffered in both groups (**Fig. 11**). The LIVE/DEAD results show that liver organoids are required to metabolize capecitabine to the active 5-FU form of the drug, inducing increased cell death in cardiac and lung organoids.

Soluble biomarkers, including urea, albumin, α-GST, IL-8, and IL-1β, were quantifed from media aliquots at days 1, 3, 5, 7, 9, 11, 13, and 15. (**Fig. 13**). Media aliquots were sent for mass spectrometry analysis to verify metabolism of capecitabine to 5-FU.

### Example 6 - 5 organoid integrated drug screening in a miniaturized microfluidic platform

A microfluidic platform having a miniaturized footprint and fluid volume was prepared and it was assessed whether the same complex drug studies are possible on this platform. Minimizing fluid volume may result in increased signal to noise ratio of pertinent biomarkers in the system circulation.

Design and Results of Experiments: Adhesive film-based microfluidic platforms were fabricated as described below. As with the full-scale platform described in Example 4, five tissue constructs were initiated containing liver, cardiac, lung, testis, and brain organoids and maintained for 7 days prior to the start of the drug study. At this point, the liver modules were removed from half of the platforms. Capecitabine (20 uM) was administered to all platforms after which viability was assessed through live/dead staining or cardiac beating behavior after 7 days of exposure (day 14 of total study).

In the miniaturized system, with liver present, capecitabine is metabolized into the toxic drug 5-FU, causing heart and lung toxicity (**Fig. 12**). Without liver, this metabolism does not occur, and cardiac and lung organoid viability does not decrease. Importantly, in this platform the brain organoid viability is high (**Fig. 12**). The LIVE/DEAD results show that liver organoids are required to metabolize capecitabine to the active 5-FU form of the drug, inducing increased cell death in cardiac and lung organoids. Moreover, brain organoids are viable, suggesting, but not wishing to be bound to any particular theory, that perhaps the scaled down volume in the system results in a better conditioned media that supports brain organoid viability. This demonstrates that the brain organoids can be maintained in this system together with the other organoid types.

Soluble biomarkers, including urea, albumin, α-GST, IL-8, and IL-1β, were quantifed from media aliquots at days 7 and 15 (**Fig. 13**). Media aliquots were sent for mass spectrometry analysis to verify metabolism of capecitabine to 5-FU.

### Miniaturized system design:

This system reduces or eliminates the surface area of PDMS exposed to media compared to other systems, such as the standard size system, thereby reducing chances for adsorption or absorption of drug compounds, toxins, soluble proteins, and secreted compounds onto or into the device walls. The device manufacturing strategy is based on tape microfluidics, laser cut PMMA, and some PDMS molding, thereby significantly minimizing the amount of PDMS surface area in contact with the media of the device. **Fig. 14** provides an overview of this fabrication methodology.

The foregoing is illustrative of the present invention, and is not to be construed as limiting thereof. The invention is defined by the following claims, with equivalents of the claims to be included therein. All publications, patent applications, patents, patent publications, sequences identified by GenBank and/or SNP accession numbers, and other references cited herein are incorporated by reference in their entireties for the teachings relevant to the sentence and/or paragraph in which the reference is presented.
The disclosure will now be further described by reference to the following numbered clauses:
Clause 1. An *in vitro* cell construct (or "organoid") useful as a tumor model, comprising:
   *(a)* a core comprised of live tumor cells; and
   *(b)* a shell surrounding (*e.g.*, encapsulating) said core, said shell comprised of live benign cells (*e.g.*, tissue cells, benign or differentiated tumor cells, etc.).
Clause 2. The construct of clause 1, wherein said live tumor cells comprise malignant cells.
Clause 3. The construct of any preceding clause, wherein said live tumor cells comprise or consist of colorectal cancer cells (*e.g.*, HCT116 cells, HT29 cells, SW480 cells, Caco2 cells, etc.), mesothelioma cancer cells (e.g., well-differentiated papillary mesothelioma cells), appendiceal cancer cells (e.g., low grade appendiceal (LGA) cells), and/or sarcoma cancer cells.
Clause 4. The construct of any preceding clause, wherein said tumor cells and said benign cells comprise mammalian cells (*e.g.*, human, mouse, rat, monkey, etc.).
Clause 5. The construct of any preceding clause, wherein said live benign cells comprise epithelial cells.
Clause 6. The construct of any preceding clause, wherein said live benign cells comprise Caco-2 cells.
Clause 7. The construct of any preceding clause, wherein said tumor cells comprise a detectable compound (*e.g.*, a fluorescent compound).
Clause 8. The construct of any preceding clause, wherein said core and/or said shell comprise a hydrogel.
Clause 9. The construct of any preceding clause grown in culture for a time of 1 or 2 days, or 1 to 2 weeks, or more.
Clause 10. An *in vitro* cell construct (or "organoid") useful as a tumor model, comprising live tumor cells from a subject.
Clause 11. The construct of clause 10, further comprising a shell surrounding (*e.g.*, encapsulating) a core of said live tumor cells, said shell comprised of live benign cells (*e.g.*, tissue cells, benign or differentiated tumor cells, etc.), said live benign cells optionally from said subject.
Clause 12. The construct of any one of clauses 10-11, wherein said live tumor cells comprise malignant cells.
Clause 13. The construct of any one of clauses 10-12, wherein said live tumor cells comprise or consist of colorectal cancer cells (*e.g.*, HCT116 cells, HT29 cells, SW480 cells, Caco2 cells, etc.), mesothelioma cancel cells (e.g., well-differentiated papillary mesothelioma cells), appendiceal cancer cells (e.g., low grade appendiceal (LGA) cells), and/or sarcoma cancer cells.
Clause 14. The construct of any one of clauses of 10-13, wherein said tumor cells and said benign cells, when present, comprise mammalian cells (*e.g.*, human, mouse, rat, monkey, etc.).
Clause 15. The construct of clause 14, wherein said live benign cells comprise epithelial cells.
Clause 16. The construct of clause 14, wherein said live benign cells comprise Caco-2 cells.
Clause 17. The construct of any one of clauses of 10-16, wherein said tumor cells comprise a detectable compound (*e.g.*, a fluorescent compound).
Clause 18. The construct of any one of clauses of 10-17, wherein said core and/or said shell comprise a hydrogel.
Clause 19. The construct of any one of clauses of 10-18, wherein said construct is grown in culture for a time of 1 or 2 days, or 1 to 2 weeks, or more.
Clause 20. The construct of any preceding clause, wherein said tumor cells and/or benign cells are prepared using a method that has a take rate of at least 50%, 60%, 70%, 80%, or 90%.
Clause 21. The construct of any preceding clause, wherein said construct comprises at least 75%, 80%, 85%, or 90% live cells based on the average number of cells in the construct at 1, 2, 3, or 4 weeks of culture.
Clause 22. A method of screening a compound of interest *in vitro* for anti-tumor activity, comprising:
   *(a)* providing at least one construct of any preceding clause;
   *(b)* contacting said compound to said construct *in vitro*; and then
   *(c)* determining the growth of said live tumor cells (*e.g.*, as compared to tumor cells of at least one like construct not contacted with said compound, and/or as compared to said live benign cells of said shell), a decrease in growth of said live tumor cells (*e.g.*, lack of proliferation of said tumor cells, death of said tumor cells, decrease in invasion of said shell by said tumor cells, etc), indicating anti-tumor activity of said compound of interest.
Clause 23. The method of clause 22, wherein said tumor cells are collected from a patient afflicted with said tumor.
Clause 24. The method of clauses 22 or 23, wherein said tumor cells are collected from a tumor in a patient.
Clause 25. The method of any one of clauses 22-24, further comprising providing at least one additional organoid (e.g., a brain, colon, lung, endothelium, cardiac, epithelial, and/or liver organoid), wherein the at least one additional organoid comprises different cells than the at least one construct.
Clause 26. The method of clause 25, wherein the at least one additional organoid comprises benign cells, optionally benign cells collected from the same patient as the tumor cells.
Clause 27. The method of clause 25 or 26, wherein the benign cells and/or the tumor cells are obtained from a biopsy from the patient and the compound is contacted to the at least one construct and/or at least one additional organoid within about 1 week after the biopsy.
Clause 28. The method of any one of clauses 22-27, wherein the at least one additional organoid is a liver organoid.
Clause 29. The method of any one of clauses 22-28, further comprising labeling the tumor cells with a detectable compound.
Clause 30. The method of any one of clauses 22-29, further comprising the step of administering said compound to said patient in a treatment-effective amount when said compound is determined to decrease the growth of said tumor cells *in vitro.*
Clause 31. The method of any one of clauses 22-30, further comprising identifying said compound of interest based on genetic screening information for said tumor cells.
Clause 32. The method of any one of clauses 22-31, further comprising performing a genetic screening analysis on said tumor cells.
Clause 33. The method of any one of clauses 22-32, wherein said compound is a prodrug.
Clause 34. A device useful for evaluating tumor cells *in vitro* (e.g., useful for evaluating metastasis of tumor cells *in vitro*, for evaluating tumor cell migration and/or invasion *in vitro*, for evaluating growth of a construct comprising tumor cells *in vitro*, and/or for evaluating response to a compound of interest), comprising:
   *(a)* a microfluidic device having a chamber, and a channel in fluid communication with said chamber;
   *(b)* a live tumor cell construct (*e.g.*, an organoid) in said chamber;
   *(c)* a growth media in said chamber and said channel;
   *(d)* a pump operatively associated with said chamber and channel and configured for circulating said media from said chamber through said channel and back to said chamber;
   *(e)* a microporous membrane (*e.g.*, a TRANSWELL^{®} microporous membrane) in said channel and positioned so that said media flows therethrough.
Clause 35. The device of clause 34, wherein said tumor cell constructs comprise a hydrogel.
Clause 36. The device of any one of clauses 34-35, wherein said tumor cell constructs consist of a single cell type.
Clause 37. The device of any one of clauses 34-36, wherein said tumor cell constructs comprise or consist of colorectal cancer cells (*e.g.*, HCT116 cells, HT29 cells, SW480 cells, Caco2 cells, etc.), mesothelioma cancel cells (e.g., well-differentiated papillary mesothelioma cells), appendiceal cancer cells (e.g., low grade appendiceal (LGA) cells), and/or sarcoma cancer cells.
38. The device of any one of clauses 34-37, wherein at least a portion of said microfluidic device is transparent, said device further comprising a detector (*e.g.*, a camera) operatively associated with said microporous membrane and configured for detecting (*e.g.*, imaging) tumor cells on said microporous membrane.
Clause 39. The device of any one of clauses 34-38, wherein the live tumor cell construct is a construct of any one of clauses 1-21.
Clause 40. A method of screening tumor cells *in vitro* for metastatic activity, comprising:
   *(a)* providing a device of any preceding clause;
   *(b)* circulating said media in said device; and
   *(c)* detecting tumor cells captured on said microporous membrane, where a greater number of tumor cells captured (*e.g.*, as compared to other tumor cells under like conditions, and/or non-metastatic cells under like conditions) indicates greater metastatic activity of said tumor cells.
Clause 41. A method of screening tumor cells *in vitro* for metastatic activity, comprising:
   *(a)* providing a device, said device including a primary chamber comprising a live tumor cell organoid and at least one secondary chamber comprising at least one organoid comprising different cells than the live tumor cell organoid;
   *(b)* circulating said media in said device; and
   *(c)* detecting tumor cells in the at least one secondary chamber, where a greater number of tumor cells present in the at least one secondary chamber (*e.g.*, as compared to other tumor cells under like conditions, and/or non-metastatic cells under like conditions) indicates greater metastatic activity of said tumor cells.42. The method of clause 41, wherein the at least one organoid is a liver, colon, brain, lung, endothelium, cardiac, or epithelial organoid.
Clause 43. The method of clause 41 or 42, wherein the at least one organoid comprises a liver organoid and an additional organoid, and wherein the liver organoid and the additional organoid are present in separate secondary chambers that are in fluid communication.
Clause 44. A method of screening a compound of interest *in vitro* for anti-metastatic and/or anti-tumor activity, comprising:
   *(a)* providing a device of any preceding clause;
   *(b)* circulating said media in said device;
   *(c)* administering said compound to said tumor cells (*e.g.*, by adding said compound to said media); and then
   *(d)* detecting tumor cells captured on said microporous membrane, where a lesser number of tumor cells captured (*e.g.*, as compared to other tumor cells under like conditions but without the administering of said compound of interest) indicates greater anti-metastatic and/or anti-tumor activity of said compound of interest.
Clause 45. A method of screening a compound of interest *in vitro* for anti-metastatic activity and/or anti-tumor activity, comprising:
   *(a)* providing a device comprising a first chamber comprising a liver organoid and a second chamber comprising a live tumor cell organoid;
   *(b)* circulating a growth medium from the first chamber to the second chamber;
   *(c)* administering a compound of interest to the liver organoid and/or the live tumor cell organoid (*e.g.*, by adding the compound to the growth medium); and
   *(d)* determining a decrease in the presence of tumor cells (e.g., a change in number or density) in the second chamber, as compared to the number of tumor cells present in the live tumor cell organoid when the test compound is not administered.
Clause 46. The method of clause 45, further comprising determining a decrease in the presence of tumor cells (e.g., a change in number or density) in the first chamber, as compared to the number of tumor cells present in the first chamber when the test compound is not administered.
Clause 47. The method of clause 45 or 46, wherein the device further comprises at least one additional chamber comprising at least one organoid having different cells than the live tumor cell organoid and liver organoid.
Clause 48. A method of screening a compound of interest *in vitro* for anti-metastatic activity and/or anti-tumor activity, comprising:
   *(a)* providing a device comprising a primary chamber comprising a live tumor cell organoid and at least one secondary chamber comprising an additional organoid including cells different than the live tumor cell organoid;
   *(b)* circulating media in the device;
   *(c)* administering the compound to the tumor cells (*e.g.*, by adding the compound to the media); and
   *(d)* determining a decrease in the presence of tumor cells (e.g., a change in number or density) in the live tumor cell organoid and/or the at least one secondary chamber, as compared to the number of tumor cells present in the live tumor cell organoid and/or the at least one secondary chamber when the test compound is not administered.
Clause 49. The method of clause 48, wherein the additional organoid is a brain, colon, lung, endothelium, cardiac, epithelial, and/or liver organoid.
Clause 50. The method of any one of clauses 40-49, wherein said tumor cells are collected from a patient afflicted with said tumor.
Clause 51. The method of clauses 40-50, further comprising the step of administering said compound to said patient in a treatment-effective amount when said compound is determined to decrease the metastasis of said tumor cells *in vitro*, decrease construct size *in vitro*, decrease the number of tumor cells *in vitro* and/or induce tumor cell death *in vitro.*

## Claims

1. An organoid, comprising:
cancer cells; and
immune system cells,
wherein said tumor cells and said live immune cells are obtained from a single subject.

2. The organoid of claim 1, wherein cancer cells are tumour cells, optionally wherein, said tumor cells are from a tumor biopsy from said single subject.

3. The organoid of claims 1 or 2, wherein said cancer cells comprise colorectal cancer cells, mesothelioma cancer cells, appendiceal cancer cells, melanoma cancer cells, glioma cancer cells, lung cancer cells, ovarian cancer cells, breast cancer cells, prostate cancer cells, liver cancer cells, and/or sarcoma cancer cells.

4. The organoid of any preceding claim, wherein said immune system cells are selected from the group consisting of dendritic cells, lymph cells, leucocytes, B cells, T cells, and any combination thereof, optionally, wherein said immune system cells are obtained from a lymph node.

5. The organoid of any preceding claim 1, wherein the organoid, grown in culture for 1 week, comprises at least 75% living cells based on the average number of cells present in the construct at 1 week of culture.

6. The organoid of any preceding claim, wherein the organoid has a diameter of about 200 µm to about 350 µm.

7. The organoid of any preceding claim, further comprising a hydrogel or a hydrogel comprising hyaluronic acid and collagen, optionally, wherein the hydrogel is a crosslinked hydrogel.

8. The organoid of claim 7, wherein the cancer cells and the immune system cells are present in the organoid in an amount of about 20 million total cells per mL of hydrogel.

9. The organoid of any preceding claim, wherein said cancer cells and/or immune system cells comprise a detectable compound.

10. The organoid of any preceding claim, wherein said organoid comprises about 1,500 to about 3,500 total cells.

11. An *in vitro* method of screening a compound of interest *in vitro* for anti-tumor activity, the method comprising:
contacting said compound to said organoid of any one of claims 1-10; and then
determining the growth of said live tumor cells, wherein a decrease in growth of said live tumor cells indicates anti-tumor activity of said compound of interest.

12. An *in vitro* method of evaluating tumor cells and/or immune cells, the method comprising:
culturing the organoid of any one of claims 1-10; and then
evaluating said live tumor cells and/or immune cells.

13. A device useful for evaluating tumor cells and/or immune cells, comprising:
a microfluidic device having a chamber and a channel in fluid communication with said chamber;
the organoid of any one of claims 1-10in said chamber;
a growth media in said chamber and said channel; and
a pump operatively associated with said chamber and channel and configured for circulating said media from said chamber through said channel and back to said chamber, optionally wherein the device, further comprises a microporous membrane in said channel and positioned so that said media flows therethrough.

14. An *in vitro* method of screening cancer cells for anti-tumor activity, comprising:
providing the device of claim 13, said device including a primary chamber comprising the construct and at least one secondary chamber comprising at least one organoid comprising cancer cells;
circulating said media in said device; and
detecting the amount of cancer cells in the at least one secondary chamber, wherein a decreased number of cancer cells present in the at least one secondary chamber indicates anti-tumor activity.

15. An *in vitro* method of screening a compound of interest for anti-metastatic and/or anti-tumor activity, comprising:
providing a device of claim 13; or
providing the device of claim 13 comprising a primary chamber comprising the organoid and at least one secondary chamber comprising an additional organoid including cells different than the tumor cells;
circulating said media in said device;
administering said compound to said construct; and then
detecting cancer cells captured on said microporous membrane, where a lesser number of cancer cells captured indicates greater anti-metastatic and/or anti-tumor activity of said compound of interest; or
determining a decrease in the presence of cancer cells in the construct and/or the at least one secondary chamber, as compared to the number of cancer cells present in the construct and/or the at least one secondary chamber when the test compound is not administered.
